(19)

Europäisches
Patentamt
European
Patent Office
Office européen
des brevets

(11) **EP 3 854 887 A1**

(12) **EUROPEAN PATENT APPLICATION**

(43) Date of publication:
**28.07.2021 Bulletin 2021/30**

(21) Application number: **20305052.1**

(22) Date of filing: **23.01.2020**

(51) Int Cl.:
*C12Q 1/6886* (2018.01)          *G01N 33/574* (2006.01)
*G16B 25/10* (2019.01)          *G16B 40/20* (2019.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Institut Jean Paoli & Irène Calmettes
13009 Marseille (FR)**
• **Ligue Nationale Contre le Cancer
75013 Paris (FR)**
• **Institut National de la Santé et de la Recherche
Médicale
75013 Paris (FR)**
• **Université d'Aix Marseille
13007 Marseille (FR)**
• **Centre National de la Recherche Scientifique
75016 Paris (FR)**

(72) Inventors:
• **TURRINI, Olivier
13007 MARSEILLE (FR)**
• **GILABERT, Marine
13008 MARSEILLE (FR)**
• **GIOVANNINI, Marc
13400 AUBAGNE (FR)**
• **NICOLLE, Rémy
94100 SAINT-MAUR-DES-FOSSÉS (FR)**
• **BLUM, Yuna
35000 RENNES (FR)**
• **IOVANNA, Juan
13008 MARSEILLE (FR)**
• **DUSETTI, Nelson
13008 MARSEILLE (FR)**

(74) Representative: **Novagraaf Technologies
Bâtiment O2
2, rue Sarah Bernhardt
CS90017
92665 Asnières-sur-Seine Cedex (FR)**

(54) **IN VITRO METHOD FOR IDENTIFYING EFFICIENT THERAPEUTIC MOLECULES FOR TREATING PANCREATIC DUCTAL ADENOCARCINOMA**

(57)     The present invention relates to in vitro methods allowing the identification of efficient therapeutic molecules for treating pancreatic ductal adenocarcinoma method, in particular to an in vitro method for determining a transcriptomic signature of sensitivity of pancreatic ductal adenocarcinoma (PDAC) tumor sample of human or animal origin to a therapeutic candidate molecule, and to an in vitro method for determining the sensitivity of PDAC cancer cells of a patient to a target molecule. The methods include a method for determining a prediction function configured to generate a sensitivity degree of a tumor sample to a candidate therapeutic molecule, wherein the tumor sample is of human or animal origin, said method comprising: obtaining (310, 340) first and second references vectors (V1i, V2i) representing respective first reference transcriptomic profiles of tumor samples of a first batch and of a second batch (B1, B2) of tumor samples of human or animal origin; obtaining (320, 350) for each tumor sample of the first and second batches (B1, B2) of tumor samples, at least one measure of sensitivity (D1i, D2i) to the candidate therapeutic molecule; performing (330) a correlation analysis to determine candidate prediction functions (PFm) by using the first reference vectors (V1i) and the measures of sensitivity (D1i) obtained for the tumor samples of the first batch of tumor samples as learning data of a set of at least one supervised or semi-supervised machine learning method, wherein each candidate prediction function (PFm) is configured to generate a sensitivity degree from a vector representing a transcriptomic profile; applying (360) at least one candidate prediction function (PFm) to each of at least one of second reference vectors to generate a predicted sensibility degree for the corresponding tumor sample of the second batch (B2) of tumor samples; and selecting (370) a candidate prediction function among the candidate prediction functions (PFm) on the basis of a comparison of the predicted sensibility degrees obtained for the tumor samples of the second batch (B2) and the measures of sensitivity obtained for the tumor samples of the second batch (B2) of tumor samples.

EP 3 854 887 A1

**(Cont. next page)**

310 Obtaining first reference vectors representative of first reference transcriptomic profiles

320 Obtaining first measures of sensitivity

330 Correlation analysis to generate candidate prediction functions

340 Obtaining second reference vectors representative of second reference transcriptomic profiles

350 Obtaining second measures of sensiitivity

360 Applying the candidate prediction functions to generate predicted sensitivity degrees

370 Accuracy assessment to select a candidate prediction function

**Figure 1**

**Description**

**Technical field**

The present invention relates to an in vitro method allowing the identification of efficient therapeutic molecules for treating pancreatic ductal adenocarcinoma method, in particular to an *in vitro* method for determining a transcriptomic signature of sensitivity of pancreatic ductal adenocarcinoma (PDAC) tumor sample of human or animal origin to a therapeutic candidate molecule, and to an *in vitro* method for determining the sensitivity of PDAC cancer cells of a patient to a target molecule.

The present invention also relates methods and algorithms suitable for use to evaluate the efficiency of an anticancer compounds for each PDAC patients or to select the most efficient anticancer compound for each PDAC patient.

The present invention relates generally to the field of transcriptomics, for effective treatment of individuals with a Pancreatic Ductal Adenocarcinoma (PDAC).

In the present document, the numbers between brackets ([ ]) refer to the List of References provided at the end of the document.

**Background of the Invention**

Pancreatic Ductal Adenocarcinoma (PDAC) is one of the most aggressive gastrointestinal tumors. An earliest genetic event in the progression of the normal ductal epithelia to premalignant pancreatic intraepithelial neoplasia (PanIN) is the mutation of the K-Ras oncogene (Deramaudt T, Rustgi AK, Mutant KRAS in the initiation of pancreatic cancer, Biochim Biophys Acta. 2005 Nov 25; 1756(2):97-101 [1]). The mutational activation of KRAS protein triggers diverse downstream effector proteins that sustain proliferation, metabolic reprogramming, anti-apoptosis, evasion of the immune response and remodeling of the microenvironment. Subsequently, chromosome rearrangements and mutations in CDKN2A, TP53, SMAD4 and other genes cause the progression from low- to high-grade PanIN and lead to PDAC (Feldmann et al., 2007 [2]; Iovanna et al., 2012 [3]; Ying et al., 2016 [4]). Tumor mass contains also cells such as the cancer-associated fibroblasts (CAFs), the T cells, the stellate cells, the macrophages, the regulatory T cells, the endothelial cells and others (Ryan Carr et al. 2016 [5]; and Pillarisetty, 2014 [6]). They play an active role in maintaining a favorable microenvironment for cancer cell survival. At molecular level, the coordination among stromal cells and between cancer and stromal cells is sustained by the exchanges of molecules (Iovanna and Closa, 2017 [7]; Leca, J. et al. JCI. 126, 1-17, 2016 [8]).

PDAC is a heterogeneous disease with a variable clinical evolution, an inconstant response to the treatments and histopathologically may present some differentiation degrees. In fact, survival after the time of diagnosis could be from 3 months to more than 5-6 years or even more, efficient response to the gemcitabine treatments is of 10% (Burris et al., 1997 [9]), whereas to folfirinox is around 30% (Conroy et al., 2011 [10]), and histologically PDAC tumors could be from very to poorly differentiate. The inventors (Nicolle et al., 2017 [11]) and others (Bailey et al., 2016 [12]) have reported that the clinical evolution, the response to the treatments as well as the differentiation degrees cannot be explained by the genetic mutations. On the contrary, the inventors demonstrated that at least the overall survival and histological characteristics of the tumors are determined by the epigenetic landscape including DNA methylation (Nicolle et al., 2017 [11]) and specific histones marks (Lomberk et al., 2018 [13]). This is conceptually important since epigenetic, contrary to the majority of the genetic mutation, is druggable and therefore modifiable.

The prediction of the clinical evolution and outcome of PDAC, as well its sensitivity to each anticancer drug, at the diagnosis time, is of evident clinical interest.

Histology is a good indicator to predicting the survival time (Lenz et al., 2011 [14]) but is it only possible in a small amount as lesser than 15% of the operable patients. While on the non-operable patients, who have a most evolved disease and represent around of 85%, the only available material is a small number of fresh cells, obtained by Endoscopic ultrasound-guided fine needle aspiration biopsy (EUS-FNA), that results not enough for determine its differentiation status. Therefore, to phenotypically characterize all the PDAC became urgent since it could be determinant for the therapeutic approach. In particular, there remains a need for in vitro or ex vivo methods for determining the short-term and long-term survival (prognosis) of said individual; or alternatively for determining the level of differentiation of PDAC in said individual.

There is to date no validated biomarkers predictive of the response to chemotherapy. MSI status, as in other solid tumors, is a predictor of response to immunotherapies based on PDL1 inhibitor. Germline BRCA1 and BRCA2 mutations were shown to be predictive of response to PARP inhibitors. dCK, hent1, and other transporters or enzymes implicated in the uptake and metabolism of the gemcitabine have also been shown to be predictive of response to gemcitabine. However, these results were challenged several times and it was demonstrated that the absence of efficient antibodies to quantify these proteins in situ was a major obstacle.

There are still no available prognostic biomarkers, or combinations of biomarkers for patients with PDAC; nor biomarkers of predictive response. Clinical heterogeneity is one of the main features of Pancreatic Adenocarcinoma (PDAC). Conventional clinico-pathological analyses such as grading are only achievable for the 15% of resectable patients and provide limited value.

Therefore, it remains a need for a more robust and clinically useful prognostic indicator applicable to all PDAC sample, and a need for *in vitro* or *ex vivo* methods for determining the prognosis of a patient with PDAC, providing an effective support for the physician for the selection of an appropriate drug for treating these patients, which includes therapeutic treatments with active principles such as gemcitabine, oxaliplatin, 5-fluorouracil (5FU), docetaxel, Irinotecan, and/or derivatives thereof.

There also remains a need for *in vitro* or *ex vivo* methods for screening compounds which are efficient for treating PDAC in a patient.

## Description of the invention

It is precisely an object of the invention to reply to these needs by providing improved *in vitro* or *ex vivo* methods for the selection of an appropriate drug for treating PDAC patients and for screening compounds which are efficient for treating PDAC in a patient.

According to a first aspect, the present invention relates to a method for determining a prediction function configured to generate a sensitivity degree of a tumor sample to a candidate therapeutic molecule, wherein the tumor sample is of human or animal origin, said method comprising:

- obtaining first reference vectors representing respective first reference transcriptomic profiles of tumor samples of a first batch of tumor samples of human or animal origin;
- obtaining for each tumor sample of the first batch of tumor samples, at least one measure of sensitivity to the candidate therapeutic molecule;
- performing a correlation analysis to determine candidate prediction functions by using the first reference vectors and the measures of sensitivity obtained for the tumor samples of the first batch of tumor samples as learning data of a set of at least one supervised or semi-supervised machine learning method, wherein each candidate prediction function is configured to generate a sensitivity degree from a vector representing a transcriptomic profile;
- obtaining second reference vectors representing respective second reference transcriptomic profiles of tumor samples of a second batch of tumor samples of human or animal origin;
- obtaining for each tumor sample of the second batch of tumor samples, at least one measure of sensitivity to the candidate therapeutic molecule;
- applying at least one candidate prediction function to each of at least one of second reference vectors to generate a predicted sensibility degree for the corresponding tumor sample of the second batch of tumor samples; and
- selecting a candidate prediction function among the candidate prediction functions on the basis of a comparison of the predicted sensibility degrees obtained for the tumor samples of the second batch and the measures of sensitivity obtained for the tumor samples of the second batch of tumor samples, wherein the selected candidate prediction function is a prediction function configured to be applied to a vector representing a transcriptomic profile of a tumor sample to generate a sensitivity degree of the tumor sample with respect to the candidate therapeutic molecule.

According to the present invention, the selected candidate prediction function (SPF) is preferably a candidate prediction function that minimizes a prediction error.

According to the present invention, the step of selecting a candidate prediction function among the candidate prediction functions may comprise selecting a candidate prediction function for which the predicted sensibility degrees obtained for the tumor samples of the second batch and the measures of sensitivity obtained for the tumor samples of the second batch of tumor samples are the most correlated.

According to the present invention, each of the set of at least one supervised or semi-supervised machine learning method is configured to:

- identify a set of gene expression components of the transcriptomic profiles represented by the first reference vectors that are correlated with the measures of sensitivity obtained for the tumor samples of the first batch of tumor samples; and
- determine at least one candidate prediction function based on the mathematical relationship between the identified set of gene expression components and the measures of sensitivity.

According to one aspect of the present invention, the set of at least one supervised or semi-supervised machine learning method may comprise a matrix factorization method allowing an identification of at least partially decorrelated matrix components, wherein determining the candidate prediction functions comprises:

- obtaining a first matrix of transcriptomic profiles from the first reference vectors;
- obtaining for the matrix factorization method at least one candidate parameter set, each of said at least one candidate parameter set including a matrix component number;
- for each of said at least one candidate parameter set, performing the correlation analysis for the concerned candidate parameter set, wherein performing the correlation analysis for the concerned candidate parameter set comprises:

  - decomposing the first matrix by the matrix factorization method allowing an identification of at least partially decorrelated matrix components, wherein the number of at least partially decorrelated matrix components is equal to the matrix component number defined in the concerned candidate parameter set;
  - identifying, among the at least partially decorrelated matrix components, a matrix component that is the most predictive component with respect to the measures of sensitivity obtained for the tumor samples of the first batch of tumor samples corresponding respectively to the first reference vectors; and
  - determining a candidate prediction function associated with the concerned candidate parameter set as a projection function on the most predictive component.

According to this aspect of the present invention, the step of applying a candidate prediction function to a second reference vector may comprise applying to the concerned second reference vector the projection function on the most predictive component to generate a predicted sensibility degree for the corresponding tumor sample of the second batch of tumor samples.

According to the present invention, the method may further comprise a step of computing a sensitivity signature to the candidate therapeutic molecule as a distribution of prediction values generated by applying the selected candidate prediction function (SPF) to at least some of the second reference vectors, wherein the orientation of the scale of the prediction values is determined on the basis of the location, on the scale of the prediction values, of the prediction values of the second reference vectors associated with the highest and / or lowest measures of sensitivity.

The present invention relates also to a method for determining a sensitivity degree of a tumor sample of a patient to one or more candidate therapeutic molecules, said method comprising the steps of:

- obtaining a vector representing a transcriptomic profile of a tumor sample of the patient;
- obtaining a prediction function configured to generate a first sensitivity degree of a tumor sample to a first candidate therapeutic molecule by the method for determining a prediction function configured to generate a sensitivity degree of a tumor sample to a candidate therapeutic molecule according to the present invention; and
- computing a sensitivity degree to the first candidate therapeutic molecule as the result of the prediction function applied to the vector representing the transcriptomic profile of the tumor sample of the patient.

The method for determining a sensitivity degree of a tumor sample of a patient to one or more candidate therapeutic molecules according to the present invention may for example comprise the steps of:

- obtaining a prediction function configured to generate at least one second sensitivity degree of a tumor sample to at least one second candidate therapeutic molecule by a method according to any of the claims 1 or 7;
- computing at least one second sensitivity degree to the respective second candidate therapeutic molecule as the result of the prediction function applied to the vector representing the transcriptomic profile of the tumor sample of the patient;
- selecting a therapeutic molecule from a set comprising the first candidate therapeutic molecule and the at least one second candidate therapeutic molecule based on the comparison between the first sensitivity degree and the at least one second sensitivity degree.

According to the present invention, each of at least one supervised or unsupervised machine learning method may be chosen from neural network methods, decision trees, k-nearest neighbors method, carrier vector machines, algorithm based on a linear model, a generalized linear model discriminant, a factor regression model, a partial least square model, a factor analysis, a support vector machine, a support vector regression, a graphical model, a tree-based model, a random forest model, a random ferns model, a naive Bayes model, a linear discriminant analysis, a quadratic linear discriminant analysis, a perceptron model, a neural network model, nearest neighbor model, a nearest prototype model, an ensemble model, a prototype-based supervised algorithm, a bagged model, a Bayesian model, a regularized linear model, a polynomial model, a rule-based model, a Gaussian process model, a mixture discriminant model, a regression spline model, a rule induction method, a prototype model, a quantile regression model, a relevance vector machine, a soft independent modelling of class analogies model, a principal component-based model, an independent-based model, a self-organizing map model.

According to the present invention, the first and / or second reference transcriptomic profile(s) of tumor sample of human origin may be obtained from patients-derived tumor xenografts, from patients-derived organoids, from patients-derived cell lines or cell culture or from patients-derived tissue.

According to the present invention the cancer may be selected from colorectal cancer, breast cancer, lung cancer, prostate cancer, melanoma, bladder cancer, kidney cancer, neuroendocrine tumors, sarcoma, head and neck cancers, liver cancer, endometrial cancer, gastric cancer, biliary tract cancer, pancreatic ductal adenocarcinoma (PDAC), the tumor sample being a tumor sample of said cancer.

According to the present invention, the cancer may be selected from pancreatic ductal adenocarcinoma (PDAC), wherein the first batch of different tumor samples of human or animal origin is a batch of different PDAC tumor samples of human or animal origin, and wherein the second batch (B2) of different tumor samples of human or animal origin is a batch of different PDAC tumor samples of human or animal origin.

As used herein, the term "sample" or "tumor sample" means any tissue tumor sample obtained and/or derived from a patient. Said tissue sample is obtained for the purpose of the *in vitro* methods of the present invention. The sample can be fresh, frozen, fixed or embedded (e.g., paraffin embedded) or derived (Patients-derived Tumor Xenografts (PDTX)). The tumor sample may result for example from the tumor resected from the patient. The tumor sample may also result from a biopsy performed in the primary tumor of the patient or performed in metastatic sample distant from the primary tumor of the patient. The sample may be obtained for example through endoscopic ultrasound-guided fine-needle aspiration (EUS-FNA) biopsy samples from patients with unresectable tumors or from tumor tissue samples from patients undergoing surgery as disclosed in Vilmann P et al. 1992 [15].

When the samples are Patients-derived Tumor Xenografts (PDTX), the samples may be obtained by any protocol known by the skilled person, for example by the protocol disclosed in document Duconseil et al., 2015 [16]. The same applies for a sample for which the sensitivity degree to a therapeutic molecule is to be determined.

According to the present invention, preferably, the samples of the first batch of different PDAC tumour sample of human or animal origin and the samples of the second batch of different PDAC tumour sample of human or animal origin are obtained by the same method.

As used herein, the term "patient" means human or animal patient.

According to the present invention, the first and second reference vectors representing respectively reference transcriptomic profiles of tumor samples of human or animal origin may be obtained by any method known by the skilled person. The methods described below are not specific to any gene expression quantification method and can be applied to any RNA-based quantification of gene expression without any specificity on processing and normalization, which includes but is not limited to: RT-qPCR, NanoString, short read Illumina sequencing often referred to as RNA-Seq, long read sequencing, microarray-technology, etc. For example, the following publications are disclosing such methods:

- Nanostring: Kulkarni MM. Digital multiplexed gene expression analysis using the NanoString nCounter system. Curr Protoc Mol Biol. 2011 Apr;Chapter 25:Unit25B.10. doi:10.1002/0471142727.mb25b10s94. PubMed PMID: 21472696 [17]
- RNA-seq and microarrays: Marioni JC, Mason CE, Mane SM, Stephens M, Gilad Y. RNA-seq: an assessment of technical reproducibility and comparison with gene expression arrays. Genome Res. 2008 Sep;18(9):1509-17. doi: 10.1101/gr.079558.108. Epub 2008 Jun 11. PubMedPMID: 18550803; PubMed Central PMCID: PMC2527709 [18].
- RNA-seq: Wang Z, Gerstein M, Snyder M. RNA-Seq: a revolutionary tool for transcriptomics. Nat Rev Genet. 2009 Jan;10(1):57-63. doi: 10.1038/nrg2484. Review. PubMed PMID: 19015660; PubMed Central PMCID: PMC2949280 [19].
- Steinbock LJ, Radenovic A. The emergence of nanopores in next-generation sequencing. Nanotechnology. 2015; 26(7):074003. doi: 10.1088/0957-4484/26/7/074003. Epub 2015 Feb 2 [20]

According to the present invention, the first and second reference vectors may be obtained by collecting for each sample the level of expression of each measured gene by obtaining the relative quantification of the corresponding mRNAs. Each gene-associated mRNA quantification, a real value, is concatenated to form a vector of real values.

According to the methods of the present invention, the reference transcriptomic profiles of tumor samples of human or animal origin may be obtained from tumor samples, from cell lines, from organoids or from patients-derived tumor xenografts, in particular for screening candidate therapeutic molecules and /or sensitivity degrees of tumor samples from donors or patients, for therapeutic and/or research purposes, in particular for the treatment of PDAC.

The present invention relates also to a computer readable medium comprising computer program instructions which when executed causes an apparatus to perform the steps of any of the methods of the present invention as defined and described herein.

The present invention relates in particular to computer readable storage medium having computer readable program instructions embodied therein, which when being loaded on a computer, one or more processors and / or a programmable hardware component in an apparatus, cause the apparatus to perform one or more steps of one or more of the methods described herein. A further embodiment may be a computer program product comprising such a computer readable storage medium. The computer readable storage medium may be non-transitory.

A computer storage medium may be any physical media that can be read, written or more generally accessed by a computer. Embodiments of a computer-readable medium includes, but are not limited to, both computer storage media and communication media including any medium that facilitates transfer of a computer program from one place to another. Specifically, computer readable program instructions to perform embodiments described herein may be stored, temporarily or permanently, in whole or in part, on a non-transitory computer readable medium of a local or remote storage device including one or more storage media.

Examples of computer storage media include, but are not limited to, a flash drive or other flash memory devices (e.g. memory keys, memory sticks, USB key drive), CD-ROM or other optical storage, DVD, magnetic disk storage or other magnetic storage devices, solid state memory, memory chip, RAM, ROM, EEPROM, smart cards, a relational database management system, a traditional database, or any other suitable medium from that can be used to carry or store computer program instructions in the form of instructions or data structures which can be read by a computer processor. Also, various forms of computer-readable medium may be used to transmit or carry instructions to a computer, including a router, gateway, server, or other transmission device, wired (coaxial cable, fiber, twisted pair, DSL cable) or wireless (infrared, radio, cellular, microwave). The computer program instructions may include code from any computer-programming language, including, but not limited to, assembly, C, C++, Basic, SQL, MySQL, HTML, PHP, Python, R, Julia, Java, Javascript, etc.

The present invention relates also to an apparatus comprising means for performing or means configured for performing the steps of any of the method of the present invention as defined and described herein.

The "means configured to perform" a given function shall be understood as functional block(s) comprising circuitry that is adapted for performing or configured to perform the given function. Moreover, any entity described herein as "means", may correspond to or be implemented as "one or more modules", "one or more devices", "one or more units", etc. Means for performing one or more functions may also comprises one or more processors and one or more memories (e.g. in a system or apparatus) for storing computer program instructions configured to, when executed by at least one processor, cause the performance (e.g. by the system or apparatus) of the one or more functions.

When provided by a processor, the functions may be provided by a single dedicated processor, by a single shared processor, or by a plurality of individual processors, some of which may be shared. Moreover, explicit use of the term "processor" may implicitly include, digital signal processor (DSP) hardware, network processor, application specific integrated circuit (ASIC), field programmable gate array (FPGA), etc. Other hardware, conventional or custom, may also be included. Their function may be carried out through the operation of program logic, through dedicated logic, through the interaction of program control and dedicated logic, or even manually, the particular technique being selectable by the implementer as more specifically understood from the context.

A memory may include a random-access memory (RAM), cache memory, non-volatile memory, backup memory (e.g., programmable or flash memories), read-only memory (ROM), a hard disk drive (HDD), a solid state drive (SSD) or any combination thereof. The ROM may be configured to store, amongst other things, an operating system of the system / apparatus and / or computer program instructions. The RAM may be used by the processor(s) for the temporary storage of data.

Other features and advantages of the invention will be apparent from the following examples, which contain additional information, exemplification and guidance that can be adapted to the practice of this invention in its various embodiments and the equivalents thereof. These examples should not be interpreted in any way as limiting the scope of the present invention.

**Brief description of the figures**

- Figure 1 shows a flowchart of an example method for determining a prediction function;
- Figure 2 shows a flowchart of an example method for determining a prediction function using a machine learning method based on a matrix factorization method;
- Figure 3 shows a data diagram that illustrates the processing steps of the method of figures 1 and 2;
- Figure 4 shows a flowchart of an example method for determining a sensitivity degree of a tumor sample of a patient to one or more candidate therapeutic molecules;

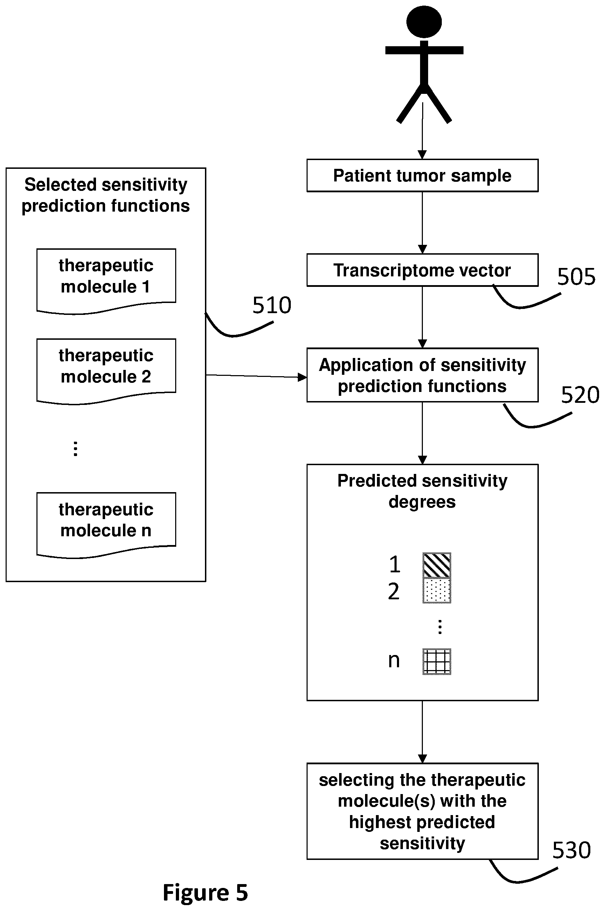

- Figure 5 shows a data processing diagram that illustrates the processing steps of the method of figure 4;
- Figure 6 shows a chemogram of a dose-response assay chemo-sensitivity in cell lines and different parameters that may be used to carry out the method of the present invention. The X-axis represents the dosage in a $\mu$M log10 scale, the y-axis represents a measure of cell counts at each dose relative to the cell count without the drug;

- Figs. 7a and 7b show the transcriptomic signatures and analysis of sensitivity to gemcitabine in cell lines;
- Figures 8a and 8b illustrate the transcriptomic signature of sensitivity to irinotecan in patient derived xenografts.
- Figure 9a and 9b illustrates the transcriptomic signature of sensitivity to irinotecan in patient derived xenografts.

It should be appreciated by those skilled in the art that any block diagrams herein represent conceptual views of illustrative circuitry embodying the principles of the invention. Similarly, it will be appreciated that any flow charts, flow diagrams, state transition diagrams, pseudo code, and the like represent various processes which may be substantially represented in computer readable medium and so executed by a computer or processor, whether or not such computer or processor is explicitly shown.

## EXAMPLES

In the different experimentations carried out, the inventors have defined the sensitivity to chemotherapeutic molecule of an important number of cell lines, obtained their whole transcriptome, identified a set of putative RNA predictive signatures associated to the sensitivity of cell lines to these molecules by using an approach based a statistical analysis method, for example, based in an Independent Component Analysis (ICA), defined the sensitivity to chemotherapeutic agents of Patients-derived Tumor Xenografts (PDTX), obtained their whole transcriptome, selected an effective signature from the set of putative RNA predictive signatures to obtain an RNA signature predictive of the response to chemotherapeutic molecules in PDAC.

The inventors have also identified a group of genes predictive of response to chemotherapeutic molecules to be used as molecular markers. Patient informed consent forms were collected and registered in a central database.

**Exemple 1:** obtaining the sensitivity to target molecules for a first set of tumor samples, cell lines

Starting from 39 different patients, as part of the PaCaOmics clinical protocol under the Paoli Calmettes Institute clinical trial "Predictive Biomarkers of Therapeutic Response in Pancreatic Tumors", multicentric clinical trial NCT01692873, 39 different samples of PDAC tumor cell lines have been obtained from PDTX. All patients presented a suspicion of PDAC and were included in the PaCaOmics clinical trial promoted by the Paoli-Calmettes Institute.

The tumor tissue used for xenograft development was deemed excess to that required for the patient's diagnosis. Two types of samples were obtained: endoscopic ultrasound-guided fine-needle aspiration (EUS-FNA) biopsy samples from patients with unresectable tumors and tumor tissue samples from patients undergoing surgery as disclosed in Vilmann P et al. 1992 [15], to cover the disease variation as large as possible. All the samples were anonymized, and postsurgical anatomopathology reports were provided for specimens from each patient.

By carrying out the protocol disclosed in document Duconseil et al., 2015 [16]), each sample obtained from EUS-FNA was mixed with 100 $\mu$L of Matrigel (BD Biosciences, Franklin Lakes, NJ) and was injected in the upper right flank of a nude immunosuppressed nude mice (Swiss Nude Mouse Crl: NU(lco)-Foxnlnu; Charles River Laboratories, Wilmington, MA). Each sample derived from surgery resection was fragmented, mixed with 100 $\mu$L of Matrigel, and implanted with a trocar (10 gauge; Innovative Research of America, Sarasota, FL) in the subcutaneous right upper flank of an anesthetized and disinfected mouse.

When the tumors reached 1 cm$^3$, the mice were sacrificed, and the tumors were removed. Xenografts that failed to develop within 6 months were discontinued. The study on animals was approved by the Animal Facility and Experimental Platform (Scientific and Technological Park of Luminy, Marseille, France).

Sensitivity to five chemotherapeutic target molecules - gemcitabine, irinotecan, oxaliplatine, docetaxel and 5FU - was determined by performing dose-response assays. The viability of cell lines was measured after being cultivated for 72h in media with increasing dose of target molecules ranging from 0.01nM to 100$\mu$M. Quantification of viable cells was performed using Cell survival was quantified using the Cell Titer-Glo assay (Promega Corporation) 3 days after adding chemo to the culture media.

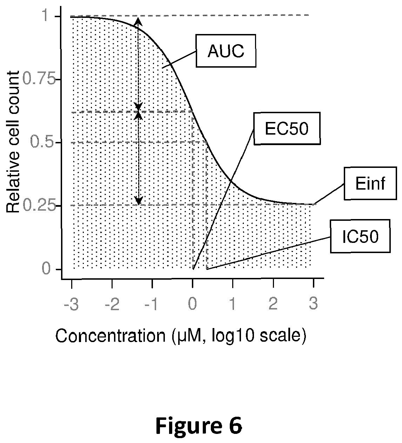

Then, the impact of chemotherapeutic treatment on each cell line was quantified using a growth rate normalized measure. Using the GRmetric R package, the number of cells at J0 (plating time) was compared to the number of cells at J3 (J3 = 3 days of cell culture) in different treatment conditions. Three growth rate normalized sensitivity metrics, as shown in annexed figure 6, were used to derive chemosensitivity signatures:

- IC50: measuring the dose at corresponding to a 50% growth inhibition;
- Einf: measuring the theoretical maximum effect (asymptotic effect);
- EC50: Efficient concentration producing 50% of the effect;
- AUC: measuring the area over the growth normalized sensitivity curve.

Each of these metrics represent different pharmacological characteristics, EC50 or IC 50 relates to potency, Einf is associated to efficacy while AUC is a global measure of the effect of the treatment.

**Example 2:** Obtention of the reference vectors representing respectively reference transcriptomic profiles of the first set of tumor samples

Total RNA was extracted from cell lines obtained as disclosed in above Example 1. PolyA enriched RNA-sequencing was performed as described herein to obtain the whole transcriptome of each cell line. Gene level count was quantified and normalized using standard methods as described herein. The invention described here is not specific to any RNA measurement technology or any particular processing steps. It is based on any measurement or surrogate measurement of gene expression levels.

Starting from each of the 39 pancreatic cell lines obtained from example 1, we have then performed the RNA-seq protocol using trizol and Guanidine Isothiocyanate and isolated with the miRneasy Mini kit (Qiagen). RNA-seq as disclosed in Lomberk et al., 2018 [13] and in Nicolle et al., 2017 [11].

Briefly, RNA expression profiles were obtained using Illumina's TrueSeq Stranded mRNA LT protocol. Sequencing followed oligo-dT capture and was done on a paired-end 100 pair flow cell. RNA libraries were prepared and sequenced by AROS Applied Biotechnology A/S (Aarhus, Denmark). RNA-seq reads were mapped using STAR 18 with the proposed ENCODE parameters, as disclosed in Dobin A, Davis CA, Schlesinger F, Drenkow J, Zaleski C, Jha S, Batut P, Chaisson M, Gingeras TR. STAR: ultrafast universal RNA-seq aligner, Bioinformatics, 2013 [21] Jan 1;29(1):15-21. doi: 10.1093/bioinformatics/bts635. Epub 2012 Oct 25. PubMed PMID: 23104886; PubMed Central PMCID: PMC3530905 [22], and SMAP on the human hg19 genome and transcript annotation (Ensembl 75), and mouse mmu38 genomes.

Gene expression profiles were obtained using FeatureCounts, for assigning sequence reads to genomic features, as disclosed in Liao Y, Smyth GK and Shi W. featureCounts: an efficient general-purpose program for assigning sequence reads to genomic features. Bioinformatics, 30(7):923-30, 2014 [23]. Only genes with at least three read counts in at least 3 samples were kept for further analysis. Gene counts were normalized using the upper-quartile approach as disclosed in Bullard et al., 2010 [23].

The next step was to identify a set of putative RNA signatures associated to the sensitivity to each target since we demonstrated that the phenotype of the PDAC is controlled by the transcriptome and epigenome (Lomberk et al., 2018 [13]; Nicolle et al., 2017 [11]).

We used for example the Independent Component Analysis (ICA) statistical method to identify in a semi-supervised manner a set of gene expression components that correlated with the sensitivity to each chemotherapeutic target molecules. This resulted in a set of candidate RNA signature predictive of the sensitivity to each chemotherapeutic target molecules.

**Exemple 3:** obtaining the sensitivity to target molecules for a second set of tumor samples, PDTX

The growth 12 PDTX as defined above was measured in mouse during treatment with each chemotherapeutic target molecule independently and under a control treatment with DMSO. The growth rate under each chemotherapeutic target molecule was compared with the control to determine the sensitivity.

Similarly to example 2, vectors representing respectively reference transcriptomic profiles of the second set of 12 PDTX tumor samples were obtained. Each RNA candidate signature predictive of the sensitivity to each chemotherapeutic target molecules was applied to the vectors representing respectively reference transcriptomic profiles of the second set of 12 PDTX tumor samples. The resulting predicted sensitivity of the 12 PDTX to the five chemotherapeutic target molecules was compared to the measured sensitivity. The candidate RNA predictive signature with the predicted sensitivity that was the closest to the observed sensitivity was selected as the final RNA signature predictive of the sensitivity to each chemotherapeutic target molecules.

Determination of a prediction function for a candidate therapeutic molecule

A method for determining a prediction function adapted to generate a sensitivity degree of a tumor sample to a candidate therapeutic molecule an RNA is be described. The tumor sample is of human origin. The prediction function is configured to be applied to a vector representing a transcriptomic profile of a tumor sample to generate a sensitivity degree of the tumor sample with respect to the given candidate therapeutic molecule.

The prediction function is determined on the basis of reference transcriptomic profiles generated for two batches B1 and B2 of tumor samples, which may be of human or animal origin.

A transcriptomic profile of tumor sample may be obtained from patients-derived tumor xenografts, from patients-derived organoids, from patients-derived cell lines or cell culture or from patients-derived tissue or using any appropriate method. Here, in the disclosed experiments, the transcriptomic profile is obtained as disclosed in example 2 above.

Each transcriptomic profile may be encoded as a data vector representing the gene expression, e.g. according to a RNA analysis of the tumor sample. The gene expression is determined with respect to a given set of genes. Each vector coefficient of a data vector representing a transcriptomic profile is equal to the number of genes obtained for a given gene of the set of genes.

It is assumed here that, for each reference transcriptomic profile corresponding to a tumor sample in any of the two batches B1 and B2 of tumor samples, a measure of sensitivity to a candidate therapeutic molecule may be obtained. Example methods for obtaining such measure of sensitivity to a candidate therapeutic molecule are disclosed herein.

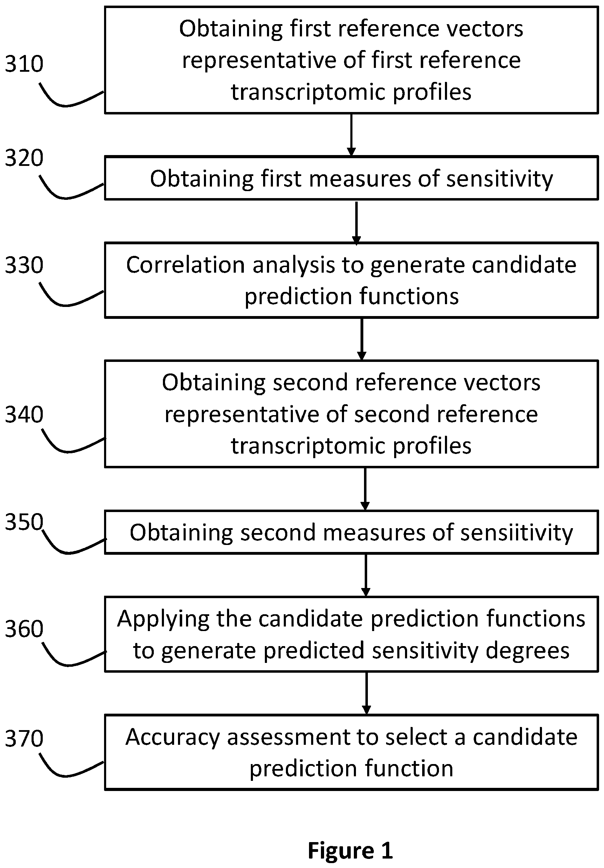

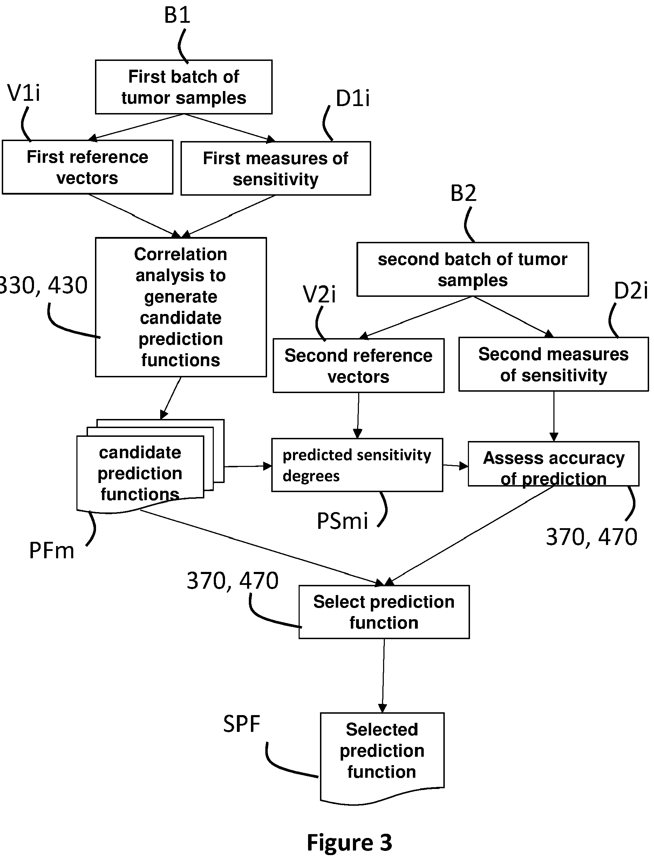

Figure 1 shows a flowchart of an example method for determining a prediction function. While the steps are described in a sequential manner, the man skilled in the art will appreciate that some steps may be omitted, combined, performed in different order and / or in parallel. Figure 3 shows a data diagram that illustrates the processing steps of the method of Figure 1.

In step 310, first reference vectors representing respective first reference transcriptomic profiles of tumor samples of the first batch B1 of tumor samples are obtained.

Let $N1$ be the number of first reference transcriptomic profiles and G be the number of genes in the set of genes for which the RNA expression was measured. Then each transcriptomic profile $i$ ($1 \leq i \leq N1$) is encoded by a vector $V1_i$ of size G*1 that may be defined as:

$$V1i = \begin{bmatrix} x1_{i,1} \\ \vdots \\ x1_{i,G} \end{bmatrix} \qquad [Eq31]$$

where j is the gene subscript varying between 1 and G.

In step 320, a measure of sensitivity of the tumor to the candidate therapeutic molecule is obtained for each first reference transcriptomic profile.

In this example, the tumor sensitivity to gemcitabine for a tumor sample of human origin is obtained from cell line cultures. It may also be obtained by using another source or method described herein or other known methods.

Let $D1_i$ ($1 \leq i \leq N1$) be the measure of sensitivity of the tumor to the candidate therapeutic molecule obtained for the transcriptomic profile encoded by the vector $V1_i$.

In step 330, a correlation analysis is performed to obtain a set of M candidate prediction functions using a set of one or more supervised or semi-supervised machine learning methods. Each of the candidate prediction functions is configured to generate a sensitivity degree from a vector representing a transcriptomic profile. Each of the candidate prediction functions is determined by using the first reference vectors $V1_i$ ($1 \leq i \leq N1$) and the corresponding measures of sensitivity $D1_i$ ($1 \leq i \leq N1$) as learning data of a supervised or semi-supervised machine learning method in order to determine one or more parameters of the concerned the candidate prediction function.

A method of the set of one or more supervised or semi-supervised machine learning methods may be a machine learning method configured to identify a set of gene expression components of the transcriptomic profiles represented by the first reference vectors that are correlated with the measures of sensitivity obtained for the tumor samples of the first batch B1 of tumor samples. One or more candidate prediction functions may be derived from the result of the correlation analysis: one or more parameters of the candidate prediction function are determined based on the mathematical relationship between the identified set of gene expression components and the measures of sensitivity.

A method of the set of one or more supervised or semi-supervised machine learning methods may be chosen from neural network methods, decision trees, k-nearest neighbors method, carrier vector machines, algorithm based on a linear model, a generalized linear model discriminant, a factor regression model, a partial least square model, a factor analysis, a support vector machine, a support vector regression, a graphical model, a tree-based model, a random forest model, a random ferns model, a naive Bayes model, a linear discriminant analysis, a quadratic linear discriminant analysis, a perceptron model, a neural network model, nearest neighbor model, a nearest prototype model, an ensemble model, a prototype-based supervised algorithm, a bagged model, a Bayesian model, a regularized linear model, a polynomial model, a rule-based model, a Gaussian process model, a mixture discriminant model, a regression spline model, a rule induction method, a prototype model, a quantile regression model, a relevance vector machine, a soft independent modelling of class analogies model, a principal component-based model, an independent-based model, a self-organizing map model.

The set of one or more supervised or semi-supervised machine learning methods may include only one machine learning method with associated distinct candidate parameter sets and / or distinct machine learning methods with respective one or more candidate parameter sets.

The candidate prediction function may be a linear function, encoded by a projection vector and / or a set of coefficients, configured to be applied a vector representing a transcriptomic profile to generate a sensitivity degree. In this case, the parameters of the candidate prediction function comprise the coefficients of the projection vector.

As the result of step 330, one or more parameters are obtained for the each of the candidate prediction functions.

In step 340, second reference vectors representing respective second reference transcriptomic profiles of tumor samples of the second batch B2 of tumor samples are obtained. Let $N2$ be the number of second reference transcriptomic profiles and G be the number of genes in the set of genes for which the RNA expression was measured. Then each transcriptomic profile $i$ ($1 \leq i \leq N2$) is encoded by a vector $V2_i$ of size G*2 that may be defined as:

$$V2i = \begin{bmatrix} x2_{i,1} \\ \vdots \\ x2_{i,G} \end{bmatrix} \qquad [Eq32]$$

where j is the gene subscript varying between 1 and G.

In step 350, a measure of sensitivity of the tumor to the candidate therapeutic molecule is obtained for each second reference transcriptomic profile. We assume here that, for each second reference transcriptomic profile in M2, the sensitivity to a therapeutic molecule is known or may be determined.

Let $D2_i$ ($1 \leq i \leq N2$) be the measure of sensitivity of the tumor to the candidate therapeutic molecule obtained for the transcriptomic profile encoded by the vector $V2_i$.

In step 360, once the parameter(s) of the each of the candidate prediction functions have been determined in step 330, each candidate prediction function is applied to each of at least one of the second reference vectors $V2_i$ to generate a predicted sensibility degree for the corresponding tumor sample of the second batch B2 of tumor samples. Let $PF_f$ be the f<sup>th</sup> candidate prediction function of the considered set of candidate prediction functions ($1 \leq f \leq L$). Then the predicted sensibility degree $PS_{fi}$ of the corresponding tumor sample of the second batch B2 of tumor samples may be computed as $PS_{fi} = PF_f(V2_i)$.

In step 370, a candidate prediction function among the L candidate prediction functions is selected on the basis of a comparison of the predicted sensibility degrees $PS_{fi}$ ($1 \leq i \leq N2$, $1 \leq f \leq L$) obtained for the tumor samples of the second batch B2 and the measures of sensitivity $D2_i$ ($1 \leq i \leq N2$) obtained for the tumor samples of the second batch B2 of tumor samples.

The selection of the candidate prediction function may be performed in various ways. The selected candidate prediction function SPF may be a candidate prediction function $PF_f$ that minimizes a prediction error. For example, the selected candidate prediction function SPF may be the candidate prediction function $PF_f$ that minimizes the sum of differences:

$$\sum_i \left( \left| PS_{fi} - D2_i \right| \right) \qquad [Eq\ 33]$$

The selected candidate prediction function SPF obtained is a prediction function specific to a given candidate therapeutic molecule and is adapted to be applied to a vector representing a transcriptomic profile of a tumor sample to generate a sensitivity degree of the tumor sample with respect to a given candidate therapeutic molecule.

Example of determination of prediction functions based on a matrix factorization method

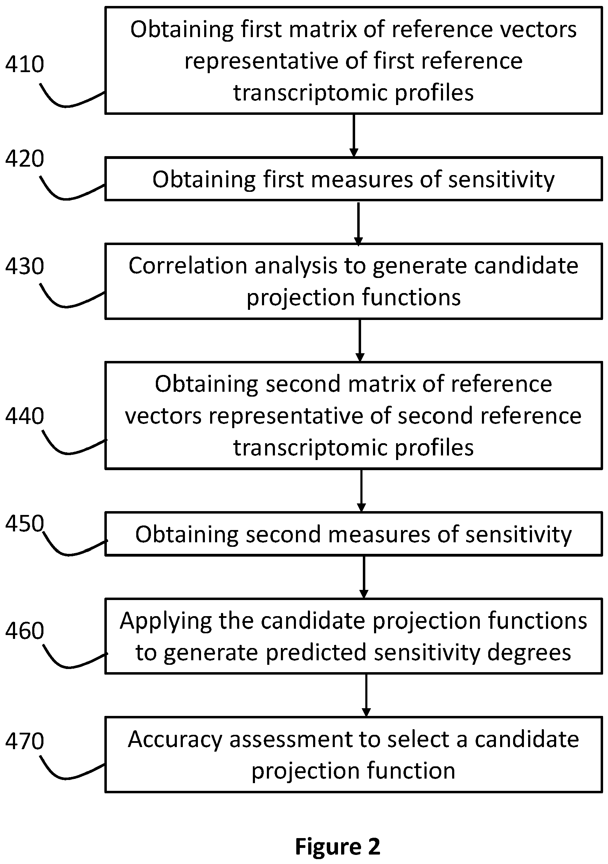

Figure 2 shows a flowchart of an example method for determining a prediction function using a machine learning method based on a matrix factorization method. Figure 3 shows a data diagram that illustrates the processing steps of the method of figure 1.

While the steps are described in a sequential manner, the man skilled in the art will appreciate that some steps may be omitted, combined, performed in different order and / or in parallel.

According to this example embodiment, only one given machine learning method is used but with distinct candidate parameter sets.

In step 410, a matrix M1 (hereafter the first reference matrix) of vectors is generated from the N1 first reference vectors representing the N1 first reference transcriptomic profiles. The size of the matrix M1 is G*N1 and the matrix M1 may be defined by

$$M1 = \begin{bmatrix} x1_{1,1} & \cdots & x1_{N1,1} \\ \vdots & \ddots & \vdots \\ x1_{1,G} & \cdots & x1_{N1,G} \end{bmatrix} \qquad\qquad [Eq41]$$

In step 420, like in step 320, a measure of sensitivity of the tumor to the candidate therapeutic molecule is obtained for each first reference transcriptomic profile.

In step 430 a correlation analysis is performed by applying a machine learning method based on a matrix factorization method allowing an identification of at least partially decorrelated components. In this example, only one machine learning method is used but with distinct candidate parameter sets to generate distinct candidate prediction functions. Thus, for a given matrix factorization method, distinct candidate parameter sets are defined.

The matrix factorization method enables to provide a new expression (hereafter the projection matrix P1i) in a new mathematical space (the projection space) of the initial matrix M1 on the basis of components that are at least partially decorrelated compared to the initial mathematical space of gene expression in which the initial matrix M1 is defined.

The matrix factorization method may be for example an independent component analysis (ICA) method, a non-negative matrix factorization method, a principal component analysis (PCA) method, a multi-dimensional scaling (MDS) method, a matrix decomposition based on eigenvalues method, a correspondence (CA) analysis method, a latent semantic analysis (LSA) method, a canonical correlation method, a factor analysis method, etc.

An example implementation using an independent component analysis (ICA) will be described in details hereafter.

After factorization, the matrix M1 may be expressed as

$$M1 \approx Qf * P1f \qquad\qquad [Eq42]$$

where Qf is a matrix (hereafter, the coefficient matrix) of size $G*Kf$ and P1f is a matrix (hereafter, the projection matrix) of size $Kf*N1$, where Kf is the number of components (hereafter, the ICA components) resulting from the factorization (e.g. the independent component analysis). Thus, the projection matrix P1f represents the projection of the N1 vectors on the Kf components resulting from the matrix factorization of the first matrix M1.

Decomposing the first reference matrix M1 by the matrix factorization method allows an identification of at least partially decorrelated matrix components, the number of at least partially decorrelated matrix components being equal to the number Kf of matrix components.

In step 430, for each candidate parameter set, the matrix M1 is factorized using a matrix factorization method.

A candidate parameter set of the matrix factorization method may include: a number Kf of matrix components for the matrix factorization and / or a subset Gf of a set of gene expression components used for representing the first reference transcriptomic profiles.

Let L be the number of the candidate parameter sets including each a combination of Kf and Gf to be tested ($1 \le f \le L$). For example L=3 candidate parameter sets are used: a first candidate parameter set includes a number K1 of matrix components equal to 3; a second candidate parameter set includes a number K2 of matrix components equal to 5 and a third candidate parameter set includes a number K3 of matrix components equal to 7. Thus the matrix M1 will be factorized three times to get three projection matrices P1f ($1 \le f \le 3$) representing the corresponding projections of the N1 vectors on the Kf components.

More generally, several projection matrix P1f are generated by varying the parameters such as the number of components Kf and / or the number (or set) of genes Gf selected among G genes. For example, the number Kf is applied between 2 and 25 ($2 \le Kf \le 25$) and Gf between 1000 and 20000 ($1000 \le K1 \le 20000$). This results in a set of L projection matrices P1f(f= 1 to L).

In step 430, the correlation analysis is performed for each projection matrices P1f as follows: each of the Kf component of the projection matrice P1f is compared to the tumor sensitivity measure to identify, for each projection matrix P1f, a component $Kf^m$ that is the most predictive of the measure of sensitivity associated with the first reference transcriptomic profiles corresponding to the tumor samples of the first batch B1 of tumor samples. The purpose is here to identify one single component $Kf^m$ for each projection matrix that allows to predict for a transcriptomic profile the sensitivity degree of the represented tumor sample to a candidate therapeutic molecule.

This identification may be performed by evaluating a statistical association between the distribution of the projections of the N1 vectors on the Kf components and the measures of sensitivity of the tumor samples to a candidate therapeutic molecule associated with the N1 vectors.

As the result of step 430, for each parameter set f(f=1 to L), a prediction function PFf is determined as a projection function P1f$^m$ on the most predictive component Kf$^m$ among all the components obtained for the L projection functions of the N1 vectors. For f = 1 to L, a projection function P1f$^m$ (or projection vector) on the Kf$^m$ component is determined: this projection function P1f$^m$ corresponds to the candidate prediction function PF$_f$ for the candidate parameter set f comprising Kf and Gf.

This identification may be performed by evaluating a statistical association between the distribution of the projections of the N1 vectors on the Kf components and the sensitivity of the tumor sample to a target therapeutic molecule associated with the N1 vectors. The evaluation of the statistical association may be performed using ANOVA (Analysis of Variance), linear models, generalized linear models, Kruskal-Wallis test, Pearson and Spearman correlation.

In an optional step, constraint(s) can be applied to discard unfit components, such as an absence of correlation with other measures of tumor proliferation. This will result in a sub-selection of L' projection functions with L' < L.

In the step 440, a second matrix M2 of transcriptomic profiles is generated from second reference vectors V2i representing respectively the second reference of transcriptomic profiles of tumor samples of the second batch B2 of tumor samples. The samples may be of human or animal origin. The size of the matrix M2 is G*N2 and the matrix M may be defined as:

$$M2 = \begin{bmatrix} x2_{1,1} & \cdots & x2_{N1,1} \\ \vdots & \ddots & \vdots \\ x2_{1,G} & \cdots & x2_{N1,G} \end{bmatrix} \qquad [Eq43]$$

In step 450, like in step 350, a measure of sensitivity of the tumor to the candidate therapeutic molecule is obtained for each second reference transcriptomic profile.

In step 460, for each candidate projection function PF$_f$ = P1f$^m$, a predicted sensibility degree for the corresponding tumor sample of the second batch B2 of tumor samples is generated by applying to the concerned second reference vector the projection function P1f$^m$ on the most predictive component K1f$^m$. In this step, L (or respectively L') projection vectors are extracted from the L (or respectively L') coefficient matrices Qf (see Eq42) and used to project the second matrix M2 on the corresponding most predictive component Kf$^m$ resulting in L (or respectively L') projected vectors P2f : P2f = P1f$^m$ * M2.

Each projected vector P2f obtained for the selected component Kf$^m$ is compared to the N2 molecular target sensitivity measures.

In step 470, an accuracy analysis is performed to select a prediction function SPF (selected projection function) among the candidate prediction functions (projection functions) P1f$^m$: the selected projection function SPF is the projection function for which the predicted sensibility degrees obtained for the tumor samples of the second batch B2 and the measures of sensitivity obtained for the tumor samples of the second batch B2 of tumor samples are the most correlated. In this step, the projection function P1f$^s$ is selected, wherein s is the value of f from 1 to L (or respectively L') for which the projected vector P2f is the most discriminant for the respectively N2 target therapeutic molecule sensitivity measures.

It should be appreciated by those skilled in the art that any supervised and semi supervised machine learning method may be used for implementing the steps 310-370 or 410-470 including the generation of L candidate prediction functions from first reference transcriptomic profiles and their respective measure of sensitivity to a therapeutic target molecule and the selection of one among the L candidate prediction functions using second reference transcriptomic profiles and their respective measure of sensitivity to the same therapeutic target molecule.

**Optimization**

In one or more embodiments, when using a matrix factorization method, the previous method steps 410-430 may be performed a first time with a first large set of genes until the coefficient matrix Q1s is obtained. Then only a reduced set of genes may be used for the determination of the prediction function at steps 440-470. For example, the genes having the highest coefficients in absolute value in the coefficient matrix Q1s may be kept for the reduced set of genes. The method steps 410-470 may be performed again with this reduced set of genes to get a corresponding coefficient matrix Q1s and projection matrix P1s for the reduced set of genes. Thus, only a reduced set of genes may be used, thereby simplifying the computations and limiting the resources that are necessary both for the RNA analysis and the computation of the matrices, projection values, etc.

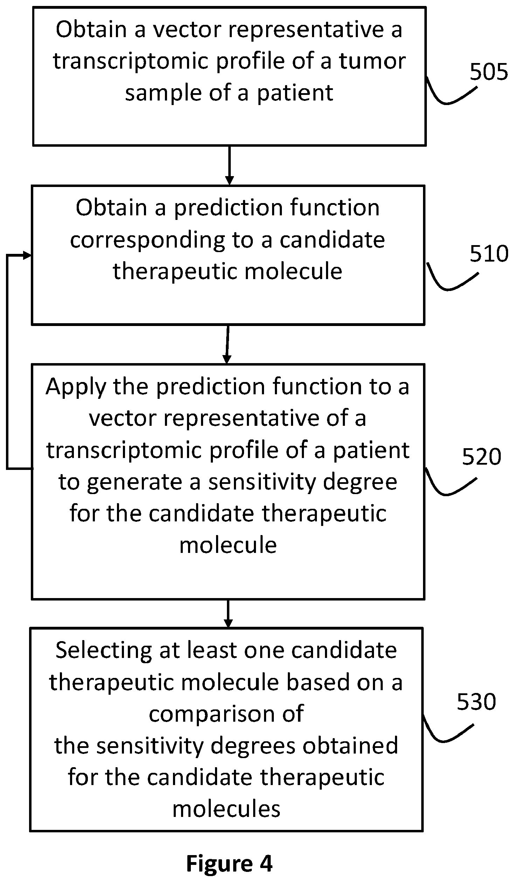

Figure 4 shows a flowchart of an example method for determining a sensitivity degree of a tumor sample of a patient to one or more candidate therapeutic molecules. While the steps are described in a sequential manner, the man skilled in the art will appreciate that some steps may be omitted, combined, performed in different order and / or in parallel. Figure shows a data processing diagram that illustrates the processing steps of the method of figure 4.

In step 505, a vector representative of a transcriptomic profile is obtained for the concerned tumor sample.

In step 510, a sensitivity degree of a tumor sample of a patient to the concerned candidate therapeutic molecule is determined, using any suitable method described herein, for example using the method described by reference to figure 1 or 2.

In step 520, the selected candidate prediction function SPF (e.g. the candidate prediction function selected in step 370 or 470) is used as prediction function to generate a sensitivity degree of the tumor sample to the candidate therapeutic molecule. A sensitivity degree to the candidate therapeutic molecule is obtained as the result of the prediction function applied to the vector representing the transcriptomic profile of the tumor sample of the patient.

The steps 510 and 520 may be repeated for at least one second candidate therapeutic molecule to obtain a sensitivity degree for each of at least one second candidate therapeutic molecule. As a consequence, for a given patient, a sensitivity degree may be obtained for each of a plurality of candidate therapeutic molecules.

In step 530, at least one candidate therapeutic molecule is selected on the basis of a comparison of the sensitivity degrees obtained each of a plurality of candidate therapeutic molecules. For example, the candidate therapeutic molecule suitable for this patient may be selected as a candidate therapeutic molecule having the highest sensitivity degree or one of the highest sensitivity degrees.

### RNA signature

A method for determining a sensitivity signature predictive of the sensitivity of one or more pancreatic ductal adenocarcinoma (PDAC) samples to a target therapeutic molecule is also disclosed.

As will be explained below, one or more specific sets of genes having the highest impact on the sensitivity degree which is determined by a prediction function for a given therapeutic molecule may be used to form a RNA signature (or sensitivity signature) predictive of the sensitivity degree. This sensitivity signature will be referred to herein as the RNA signature. The number of genes in the RNA signature may be lower than 100, for example lower than 50 genes or lower than 20 genes.

A sensitivity signature to the candidate therapeutic molecule or RNA signature for the candidate therapeutic molecule may be computed based on the candidate prediction function selected at step 370 and /or step 470. The RNA signature may be computed as a distribution of prediction values generated by applying to each of the second reference vectors the selected candidate prediction function SPF obtained at step 370 and /or step 470.

For example, when the candidate prediction functions are candidate projection functions (see steps 410-470 described by reference to figure 2) the RNA sensitivity signature is computed as a distribution of projection values of the N2 vectors on the Ksm component of the P2s projection function.

The sensitivity degrees of the distribution may be scaled to be defined on a range of easily interpretable values, for instance between 0 and 1 to be expressed as percentage or normalized around zero with zero mean and unit standard deviation.

To be able to determine which values of the distribution correspond to the higher (or respectively lower) sensitivity, a correspondence between the sensitivity measures and a distribution of prediction values of vectors representing reference transcriptomic profiles belonging is taken into account for determining the orientation of the prediction value scale. The orientation of the scale of the prediction values is determined on the basis of the location, on the scale of the prediction values, of the prediction values of one or more second reference vectors associated with the highest and / or lowest measures of sensitivity.

The scale of prediction values may be normalized to facilitate interpretation of the prediction values. For example, to get a normalized scale between -1 and +1 or between 0 and 1, each prediction value is normalized with respect to the mean value and / or lowest and highest values and / or standard deviation of the distribution. The scale of prediction values may more generally be a defined by its upper and lower values such as [0, 100], [-100; +100], [0; 1] [-1, +1], etc.

The scaling function may be defined in different manner. For example, a normalized score nsc may computed from a non-normalized value sc, the mean m and standard deviation $\sigma$ of the distribution corresponding to the reference RNA sensitivity signature. In an example, the standard deviation $\sigma$ is multiplied by a real number w (scaling coefficient) that may be set between 1 and 3 ($1 \leq w \leq 3$). The scaling function is for example

$$\mathrm{nsc} = \frac{sc - m}{w \cdot \sigma} \qquad\qquad [\mathrm{Eq}51]$$

The RNA sensitivity signature enables to represent the degree of sensitivity (or level of sensitivity) to a target molecular therapy associated with a transcriptomic profile using a single numerical value defined relatively to a scale of values. A continuous scale or a scale of discrete values may be used.

The sensitivity degree to a target molecular therapy may be computed for any transcriptomic profile.

For example, the gene expression is an RNA expression and the specific set of genes, as defined by the HUGO Gene Nomenclature Committee, includes part or all of the following genes:
MIR205HG, C20orf197, IGFBP1, FAT2, NIPAL4, CIDEC, CSTA, TMEM37, PDE11A, PBX1, PVRL1, CTGF, GSTM4, TIMP2, RASSF4, ASTN2, C22orf23, MICALL1, TRAF3IP2, HAS3, KLHL41, ZCWPW2, TCOF1, RCC1, TRAP1, FBXO9, DAP, CD63, NOB1, RUVBL1

**Exemple 4**: Example of sensitivity signatures obtained by the present invention, by carrying out the process as disclosed in the above examples

Example of a reduced RNA signature for gemcitabine

For example, the RNA signature for gemcitabine is expressed using a specific set of genes including part or all of the genes of a reduced set of genes.
For gemcitabine the identified reduced set of genes includes: MIR205HG, IGFBP1, FAT2, GAL, FGFR3, PARD3B, MAOB, CEL, PLA2G16, TMEM37, COL28A1, LRRC66, PDE11A, PBX1, C12orf54, DDN, NXPE1, MSI2, JUN, CHCHD6, C11orf84, MICU2, RCC1, FBXO9, NOB1, PRRC2B, CTNNB1, RPL5, RUVBL1, MANBAL.
For gemcitabine the identified reduced set of genes includes: KRT5, GAL, C4BPB, CREB3L1, PLAC8, SLC40A1, MAOB, RNF144B, RNASE7, ADAD2, CAPN14, PLA2G16, LYPD6, PVRL3, SH2D3C, DDN, SRC, THSD7B, SNED1, FBL, MSI2, JUN, SOCS6, C11orf84, TRAK2, MICU2, LYAR, PRRC2B, RPL5, ALG5.
For gemcitabine the identified reduced set of genes includes: EPS8L3, TNFRSF11B, VNN1, TSLP, ADD2, PLAC8, NDP, S100A4, RNASE7, ADAD2, CAPN14, FRY, LYPD6, CXCL3, CACNA2D4, PVRL3, SPRR2E, SLC7A5, SPTB, KRT2, SH2D3C, SRC, CDKN2C, TRAK2, PRKAB1, STX6, RABEPK, EIF2A, ALG5, ZFP64.

Example of a reduced 5-fluorouracil signature

For 5-fluorouracil, the RNA signature is expressed using a specific set of genes including part or all of the genes of a reduced set of genes.
For 5-fluorouracil the identified reduced set of genes includes: BVES, VCAM1, RNF150, EDAR, FXYD3, NFE4, PTPRZ1, C1orf106, SLC7A2, HEG1, RASEF, KLF17, CLCNKA, ATP8B1, HIPK2, NRP2, PDS5B, FBXL13, FERMT1, USP12, CDKN2C, PLBD2, MAGED1, ACP6, MGST2, TASP1, ITPA, ZFYVE1, PSMD2, RAC1.
For 5-fluorouracil the identified reduced set of genes includes: BVES, ZBED2, ANGPT1, LEMD1, PTPRZ1, C1orf106, SLC7A2, HEG1, RAB39B, LGI2, USP43, RASEF, KLF17, AKR1B1, HSPB7, EPS8L2, NRP2, MFGE8, ITGA6, STON2, IL17RE, DACT3, GRTP1, GABARAPL1, MGST1, FKBP1B, ANXA1, PLBD2, ACP6, USP5.
For 5-fluorouracil the identified reduced set of genes includes: CEACAM5, SRGN, LEMD1, KIF5C, RAB39B, SHISA3, CLDN7, LGI2, UNC5B, CYP3A5, HSPB7, CYP2W1, ANO9, N4BP2L1, C9orf170, ELOVL3, DIRAS1, ITGA6, SH3RF2, STON2, GRTP1, GABARAPL1, ANXA1, NET1, ETHE1, MAP3K3, ATN1, WBP11, WIPI1, USP5.
For 5-fluorouracil the identified reduced set of genes includes: AOC1, VCAM1, FXYD3, NFE4, ZNF385B, VSNL1, BRCA2, TMEM26, TPST1, NHLRC3, PROSER1, PDS5B, N4BP2L2, FERMT1, SMARCC1, STK24, PSD4, USP12, CPEB4, CDKN2C, ITSN1, PDE4DIP, MAGED1, GSTO2, CALU, P4HA3, CCDC77, ITPA, PSMD2, SLC41A1

Example of a reduced irinotecan signature

For irinotecan, the RNA signature is expressed using a specific set of genes including part or all of the genes of a reduced set of genes.
For irinotecan the identified reduced set of genes includes: TFF2, MLPH, LFNG, SLC16A3, GPC1, LDLRAD2, TFEB, HSPG2, IRS1, PC, NPAS2, METRNL, CHPF, HPCAL1, RGS10, SLC38A1, POU2F1, PRADC1, EIF4B, NPM1, RPAP3, RPS15A, IPO7, EIF2A, TAF1D, TAF1B, PFDN4, SNHG1, NACA, TFAM.
For irinotecan the identified reduced set of genes includes: TNS4, LFNG, SLC16A3, DUSP4, GPC1, AHR, TFEB, ITGB5, HPCAL1, RGS10, EPHA2, EPHX1, POU2F1, RABAC1, STOML1, STAG2, CCNJ, DDX21, BCL2L1, CRELD2, IQCB1, ARID2, IARS, RPS24, RPS15A, DKC1, CSNK2A2, PFDN4, TFAM, GAR1.
For irinotecan the identified reduced set of genes includes: PSCA, ALOX5, HPGD, TIMP1, TMEM105, DUSP4, SMPD1, LONRF1, EPHX1, S100A6, XPOT, RABAC1, STOML1, STAG2, CCNJ, BCL2L1, ARID2, MYEOV2, THG1L, PIP5K1C, LETMD1, DKC1, CUL1, ST13, U2SURP, GAR1, RPL22, OST4, RPL6, RNF41.
For irinotecan the identified reduced set of genes includes: VSTM2L, PCDH7, MLPH, FOXQ1, ZDHHC22, SH3BP4, LDLRAD2, IRS1, PC, NPAS2, ABCA7, ITM2C, PLXNB2, RHOB, INF2, SLC38A1, PGAP3, NAP1L1, GRPEL2, EIF4B, LTA4H, RPAP3, LRPPRC, EIF2A, TAF1B, RPL5, HNRNPA1, EIF3M, SUPV3L1, EIF2S2.

Example of a reduced docetaxel signature

For docetaxel, the RNA signature is expressed using a specific set of genes including part or all of the genes of a reduced set of genes.

For docetaxel the identified reduced set of genes includes: GPR1, SELPLG, CAPN14, TIMP1, NRARP, EPB41L4A-AS2, FZD1, IRX5, DDN, PON2, SRC, KAZN, C3orf18, AMDHD1, MTL5, PROX2, TMEM159, CPNE2, RRP36, ELK4, NSF, DCAF10, CTBP1-AS2, RPS7, CLNS1A, MSL2, RPL11, TM9SF1, RNF25, DPY30.

For docetaxel the identified reduced set of genes includes: FAM65B, SSTR1, PLAU, CAPN14, SLC2A5, PBX1, NRARP, IL2RB, FAM111B, GCSAM, PON2, AMDHD1, TMEM159, FAM60A, RRP36, PABPC1, ACAA1, SLC25A36, SORD, RPS3, MYEOV2, FOXK1, NSF, OSBPL2, MSL2, IP6K1, ARPC4, TM9SF1, ATP6AP1, DPY30.

For docetaxel the identified reduced set of genes includes: FAM65B, CNR1, IGFL2, PNMT, VSTM2L, SLC13A3, SLC2A5, GUCY1B2, HOXD8, GLIPR2, CHN2, ABLIM3, GBX1, GCSAM, E2F2, FRMD4A, FAM60A, ACAA1, SORD, FOXK1, GMPPB, RPS27A, RPL24, OSBPL2, RPL31, IP6K1, EIF2S2, NDUFB1, ATP6AP1, CPSF3.

For docetaxel the identified reduced set of genes includes: DCN, RBP4, GPR1, MAOB, AIF1L, TLE6, EPB41L4A-AS2, FSTL3, IRX5, FLVCR2, RPS6KA5, DDN, CITED2, SRC, MTL5, CYB561D2, MTHFD2, SIAH2, DCAF10, CTBP1-AS2, GYG1, ACP2, EIF2A, RPS7, BZW2, CCT4, RNF25, EMC3, COX7A2L, LCMT1

Example of a reduced oxaliplatin signature

For oxaliplatine, the RNA signature is expressed using a specific set of genes including part or all of the genes of a reduced set of genes.

For oxaliplatin the identified reduced set of genes includes: SLC13A3, NOS3, RAMP2, ASNS, OPTC, SMAGP, FASN, RGS10, PERI, FRMD4A, MLLT1, GFPT1, ACVR2B, RPTOR, LRPAP1, MYEOV2, BCOR, P4HB, DCAF10, DCLRE1C, VPS18, NSMCE4A, NT5C3A, ANKRD54, GDI2, BCS1L, RNF40, EMC3, PI4KB, SMIM12.

For oxaliplatin the identified reduced set of genes includes: FN1, FAM65B, CPE, COLCA1, SLC13A3, RAMP2, ADCY4, SMPD1, C17orf78, LPXN, STX3, MLLT1, LRPAP1, MYEOV2, BCOR, SLC39A3, LMAN2, DCAF10, DHX32, IL17RA, FNBP4, MGEA5, CSNK1D, NT5C3A, OS9, GDI2, BZW2, EIF2S2, L3MBTL2, EMC3.

For oxaliplatin the identified reduced set of genes includes: ROS1, FN1, CPE, FZD1, GSDMB, AREG, SMPD1, C17orf78, INHBC, PRELID2, TRAF5, LPXN, TTC39B, RPIA, CEBPG, LTA4H, PIP5K1C, GARS, R3HCC1, SLC39A3, LMAN2, TAF1D, ZNF142, DHX32, LRFN3, MUL1, MGEA5, MRPS7, EIF2S2, RNF25.

For oxaliplatin the identified reduced set of genes includes: LOXL1, BEND7, NOS3, EPB41L4A-AS2, ASNS, OPTC, OSTN, GPT2, RGS10, FRMD4A, SLC35D2, TMEM104, SLC4A5, CRELD2, RPTOR, PPP5C, CLPTM1, E2F5, P4HB, MTHFD2, MKL1, TIAL1, ALG12, ANKRD54, BCS1L, TM9SF1, RBM17, IPO13, SLC25A44, SMIM12.

Others sets of genes allow to validate the method of the present invention as defined in the annexed claims throughout numerous experiments carried out by the inventors. Without limitation of the scope of the invention, the inventors provide here some examples of other sets of genes allowing the generation of a RNA signature as defined in the present invention.

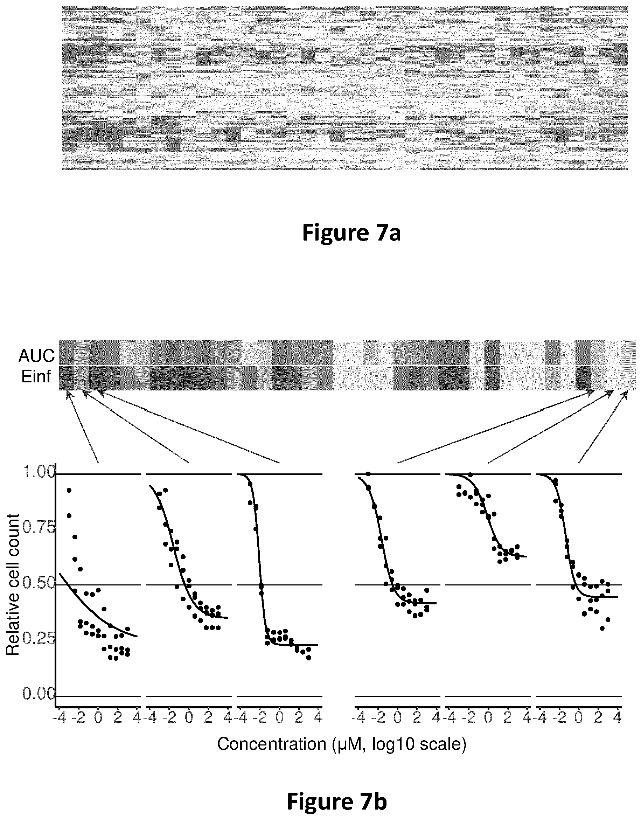

Annexed figures 7a and 7b show the transcriptomic signature of sensitivity to gemcitabine in 38 cell lines.

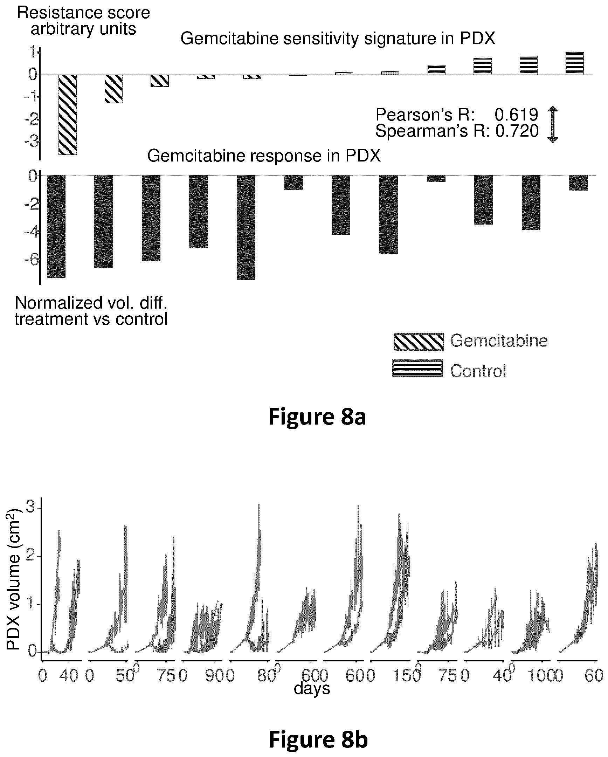

Annexed figures 8a and 8b illustrate the transcriptomic signature of sensitivity to gemcitabine in 12 patient derived xenografts. The 12 PDX are ordered by the score given by the gemcitabine signature on the transcriptomic profiles of the PDX. The figure also shows the sensitivity of PDX to gemcitabine (ordered by the signature) using the normalized volume difference between the gemcitabine-treated and control PDX and the associated growth rates.

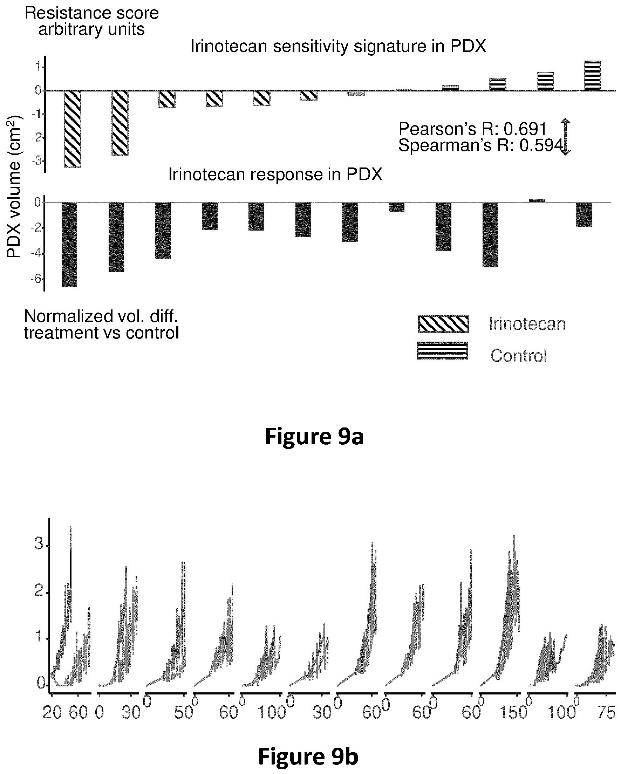

Annexed figures 9a and 9b illustrate the transcriptomic signature of sensitivity to irinotecan in 12 patient derived xenografts. The 12 PDX are ordered by the score given by the irinotecan signature on the transcriptomic profiles of the PDX. The figure also shows the sensitivity of PDX to irinotecan (ordered by the signature) using the normalized volume difference between the irinotecan-treated and control PDX and the associated growth rates.

**Example 5:** Additional example of determining the chemosensitivity of PDAC by Transcriptomics

38 pancreatic adenocarcinoma cell lines were derived from PDTX as above disclosed reported (see also Duconseil et al., 2015). Total RNA was extracted from these cell lines. PolyA enriched RNA-sequencing was performed as described above to obtain the whole transcriptome of each cell line. Gene level count was quantified and normalized using standard methods as described above. The invention described here is not specific to any RNA measurement technology or any particular processing steps. It is based on any measurement or surrogate measurement of gene expression levels.

**Chemograms**

Each cell line was treated with gemcitabine, irinotecan, oxaliplatin, docetaxel or 5FU with varying dosage from 0.001 $\mu$M to 1000 $\mu$M. Cell survival was quantified using the Cell Titer-Glo assay (Promega Corporation) 3 days after adding chemo to the culture media.

Then, the impact of chemotherapeutic treatment on each cell line was quantified using a growth rate normalized measure. Using the GRmetric R package, the number of cells at J0 (plating time) was compared to the number of cells at J3 in different treatment conditions. Three growth rate normalized sensitivity metrics were used to derive chemo sensitivity signatures, as represented in figure 6.

Cell lines were ordered by these metric for each chemotherapy dose response assay. For each chemotherapy and each metric, the three most sensitive (lowest Einf, lowest IC50 and highest AUC) and the three most resistant cell lines were selected. For these 18 comparisons (3 metrics x 6), the N most differential genes were selected using a mean-variance relationship-based precision weight for a moderate t-test. The sets of N selected genes are hereafter referred to as the

**Chemosensitivity transcriptomic signatures.**

Different cell lines were ordered by these metrics for each chemotherapy dose response assay. For each chemotherapy and each metric, all cell lines ranked as the k most sensitive (lowest Einf, lowest IC50 and highest AUC) and the k most resistant were selected. For these 18 comparisons (3 metrics x 6 chemo), genes were ordered from the most differential to the least differential between the resistant versus sensitive cell using a mean-variance relationship-based precision weight for a moderate t-test. From this ranked list of genes, only those with a log2 Fold-Change superior to t in absolute value are kept. All sets of potential predictive genes were generated by selecting the n top ranked genes with a t threshold in a k cell lines comparison. The combinations of all k, t, and n parameters was used with:

- k: {3, 4}
- t: {1, 2}
- n: [2, 60]

This resulted in the generation of 236 gene sets per drug-metric pairs. The set of most predictive genes for each of the 18 comparisons, were selected based on the combination of criteria: the accuracy of classification into resistant or sensitive in a 10-times repeated 3-fold cross-validation using the gene expression of the potential gene set to evaluate, the spearman correlation between the probability of being classified as sensitive and the actual metric for all cell lines (not only those considered as resistant or sensitive), and the R2 of a regression model using all the cell lines and the gene expression values of the potential gene set to evaluate in a 10-times repeated 3-fold cross-validation setting. Gene sets were ranked for these three criteria in each of the 18 drug-metric pair comparison and the gene set with the best mean rank was selected. This resulted in 18 sets of genes for each metric-drug pair, which are hereafter referred to as the chemosensitivity transcriptomic signatures.

**Prediction of the chemosensitivity by the transcriptomic signatures**

To predict the sensitivity of a novel sample for a given chemotherapy, e.g. from a patient tumor sample or a pre-clinical model, the chemosensitivity transcriptomic signatures are used to derive a supervised binary classifier from the transcriptomic profiles of the 3 sensitive and 3 resistant cell lines. The supervised classifier is trained on the 6 cell lines using the gene expression levels of the associated chemosensitivity transcriptomic signatures as the explanatory variables X used to predict the dependent binary variable Y encoded as 0 for resistant cell lines and 1 for sensitive cell lines. Any binary classification model can be used for this task, including but not limited to: Support Vector Machine, Random Forest, Linear or Quadratric Discriminant Analysis, Generalized linear models, Penalized linear models, k-nearest neighbor, nearest centroid, etc. Regression methods can also be applied directly on the growth rate normalized sensitivity metrics. In these cases, the explanatory variables X are composed of the gene expression values of the chemosensitivity signatures in all cell lines (instead of the 3 sensitive vs 3 resistant) and the dependent variable Y is the appropriate growth rate normalized sensitivity metric. In this case, any supervised or semi-supervised regression model can be used, including but not limited to: Support Vector Regression, Random Forest, Generalized linear models, Penalized linear models, matrix factorization approaches, etc. This step results in a task-specific prediction algorithm obtain from the training of a supervised binary classification, a supervised regression or a semi-supervised model. Hereafter referred to as the predicting model or more simply the model. Trained on the cell lines gene expression values, the model can be applied in a new sample for which the necessary gene expression values X are known, the model will output a value $\hat{Y}$, either a probability of being sensitive for binary classification models or a prediction of the growth rate normalized sensitivity metric for supervised regression and semi-supervised models. This value $\hat{Y}$ informs directly on the sensitivity and the type of sensitivity (potency, efficacy or global) of a sample to the chemotherapeutic agent associated to the signature used.

This procedure can be applied to any PDAC gene expression profiles providing an $\hat{Y}$ value for each of the chemotherapeutic sensitivity signatures.

**List of References**

[1] Deramaudt T, Rustgi AK, Mutant KRAS in the initiation of pancreatic cancer, Biochim Biophys Acta. 2005 Nov 25; 1756(2):97-101.

[2] Feldmann et al., Molecular genetics of pancreatic intraepithelial neoplasia, J Hepatobiliary Pancreat Surg. 2007; 14(3):224-32. Epub 2007 May 29.

[3] Iovanna et al., Current knowledge on pancreatic cancer. Front Oncol. 2012; 2:6. doi: 10.3389/fonc.2012.00006. eCollection 2012.

[4] Ying et al., Genetics and biology of pancreatic ductal adenocarcinoma. Genes Dev. 2016; 30(4):355-85. doi: 10.1101/gad.275776.115.

[5] Ryan Carr and Martin E. Fernandez-Zapico, Pancreatic cancer microenvironment, to target or not to target?, EMBO Mol Med. 2016 Feb; 8(2): 80-82.

[6] Venu G Pillarisetty, The pancreatic cancer microenvironment: an immunologic battleground, OncoImmunology 3:8, e950171; August 1, 2014.

[7] J. Iovanna and Closa, "Factors released by the tumor far microenvironment are decisive for pancreatic adenocarcinoma development and progression", Oncoimmunology, 2017 Aug 8;6(11).

[8] Leca, J. et al. Cancer-associated fibroblast-derived annexin A6+ extracellular vesicles support pancreatic cancer aggressiveness. J Clin Invest. 2016; 126(11):4140-4156. doi: 10.1172/JCI87734. Epub 2016 Oct 4.

[9] HA Burris et al., Improvements in survival and clinical benefit with gemcitabine as first-line therapy for patients with advanced pancreas cancer: a randomized trial, J Clin Oncol. 1997 Jun;15(6):2403-13.

[10] Thierry Conroy et al., FOLFIRINOX versus Gemcitabine for Metastatic Pancreatic Cancer, New England Journal of Medicine, 2011; 364:1817-1825 / DOI: 10.1056/NEJMoa1011923.

[11] Nicolle et al., Pancreatic Adenocarcinoma Therapeutic Targets Revealed by Tumor-Stroma Cross-Talk Analyses in Patient-Derived Xenografts, Cell Rep. 2017 Nov 28;21(9):2458-2470.

[12] Bailey P et al. Australian Pancreatic Cancer Genome Initiative. Genomic analyses identify molecular subtypes of pancreatic cancer; Nature 2016;531:47-52.

[13] Gwen Lomberk et al., Distinct epigenetic landscapes underlie the pathobiology of pancreatic cancer subtypes,, NATURE COMMUNICATIONS (2018) 9:1978, DOI: 10.1038/s41467-018-04383-6 / www.nature.com/naturecommunications.

[14] Jiri Lenz et al., Clinicopathological correlations of nestin expression in surgically resectable pancreatic cancer including an analysis of perineural invasion; Journal of gastrointestinal and liver diseases: JGLD. 20(4), 389-396 (2011).

[15] P. Vilmann et al., Endoscopic ultrasonography with guided fine needle aspiration biopsy in pancreatic disease, Gastrointest Endosc. 1992 Mar-Apr; 38(2):172-3.

[16] Pauline Duconseil et al., Transcriptomic Analysis Predicts Survival and Sensitivity to Anticancer Drugs of Patients with a Pancreatic Adenocarcinoma, The American Journal of Pathology, Vol. 185, No. 4, April 2015.

[17] Kulkarni MM Digital multiplexed gene expression analysis using the NanoString nCounter system. Curr Protoc Mol Biol. 2011 Apr;Chapter 25:Unit25B.10. doi:10.1002/0471142727.mb25b10s94. PubMed PMID: 21472696.

[18] Marioni JC, Mason CE, Mane SM, Stephens M, Gilad Y. RNA-seq: an assessment of technical reproducibility and comparison with gene expression arrays. Genome Res. 2008 Sep;18(9):1509-17. doi: 10.1101/gr.079558.108. Epub 2008 Jun 11. PubMedPMID: 18550803; PubMed Central PMCID: PMC2527709.

[19] Wang Z, Gerstein M, Snyder M. RNA-Seq: a revolutionary tool for transcriptomics. Nat Rev Genet. 2009 Jan;10(1):57-63. doi: 10.1038/nrg2484. Review. PubMed PMID: 19015660; PubMed Central PMCID: PMC2949280.

[20] Steinbock LJ, Radenovic A. The emergence of nanopores in next-generation sequencing. Nanotechnology. 2015; 26(7):074003. doi: 10.1088/0957-4484/26/7/074003. Epub 2015 Feb 2.

[21] Dobin A, Davis CA, Schlesinger F, Drenkow J, Zaleski C, Jha S, Batut P, Chaisson M, Gingeras TR. STAR: ultrafast universal RNA-seq aligner, Bioinformatics, 2013.

[22] Jan 1;29(1):15-21. doi: 10.1093/bioinformatics/bts635. Epub 2012 Oct 25. PubMed PMID: 23104886; PubMed Central PMCID: PMC3530905. Liao Y, Smyth GK and Shi W. featureCounts: an efficient general-purpose program for assigning sequence reads to genomic features. Bioinformatics, 30(7):923-30, 2014

**[23]** Bullard et al., Evaluation of statistical methods for normalization and differential expression in mRNA-Seq experiments, BMC Bioinformatics 2010 11:94 / http://www.biomedcentral.com/1471-2105/11/94.

**[24]** Moffitt RA, Marayati R, Flate EL, et al: Virtual microdissection identifies distinct tumor- and stroma-specific subtypes of pancreatic ductal adenocarcinoma. Nat Genet 47:1168-1178, 2015

**[25]** Maurer HC, Holmstrom SR, He J, et al: Experimental microdissection enables functional harmonisation of pancreatic cancer subtypes. Gut , 2019

**Claims**

1. Method for determining a prediction function configured to generate a sensitivity degree of a tumor sample to a candidate therapeutic molecule, wherein the tumor sample is of human or animal origin, said method comprising:

   - obtaining (310) first reference vectors (V1i) representing respective first reference transcriptomic profiles of tumor samples of a first batch (B1) of tumor samples of human or animal origin;
   - obtaining (320) for each tumor sample of the first batch (B1) of tumor samples, at least one measure of sensitivity (D1i) to the candidate therapeutic molecule;
   - performing (330) a correlation analysis to determine candidate prediction functions ($PF_m$) by using the first reference vectors (V1i) and the measures of sensitivity (D1i) obtained for the tumor samples of the first batch of tumor samples as learning data of a set of at least one supervised or semi-supervised machine learning method, wherein each candidate prediction function ($PF_m$) is configured to generate a sensitivity degree from a vector representing a transcriptomic profile;
   - obtaining (340) second reference vectors (V2i) representing respective second reference transcriptomic profiles of tumor samples of a second batch (B2) of tumor samples of human or animal origin;
   - obtaining (350) for each tumor sample of the second batch (B2) of tumor samples, at least one measure of sensitivity (D2i) to the candidate therapeutic molecule;
   - applying (360) at least one candidate prediction function ($PF_m$) to each of at least one of second reference vectors to generate a predicted sensibility degree for the corresponding tumor sample of the second batch (B2) of tumor samples; and
   - selecting (370) a candidate prediction function among the candidate prediction functions ($PF_m$) on the basis of a comparison of the predicted sensibility degrees obtained for the tumor samples of the second batch (B2) and the measures of sensitivity obtained for the tumor samples of the second batch (B2) of tumor samples,

   wherein the selected candidate prediction function (SPF) is a prediction function configured to be applied to a vector representing a transcriptomic profile of a tumor sample to generate a sensitivity degree of the tumor sample with respect to the candidate therapeutic molecule.

2. The method according to claim 1, wherein the selected candidate prediction function (SPF) is a candidate prediction function that minimizes a prediction error.

3. The method according to claim 1 or 2, wherein selecting a candidate prediction function among the candidate prediction functions comprises selecting (470) a candidate prediction function for which the predicted sensibility degrees obtained for the tumor samples of the second batch (B2) and the measures of sensitivity obtained for the tumor samples of the second batch (B2) of tumor samples are the most correlated.

4. The method according to any of claims 1 to 3, wherein each if the set of at least one supervised or semi-supervised machine learning method is configured to

   - identify a set of gene expression components of the transcriptomic profiles represented by the first reference vectors that are correlated with the measures of sensitivity obtained for the tumor samples of the first batch (B1) of tumor samples and
   - determine at least one candidate prediction function based on the mathematical relationship between the identified set of gene expression components and the measures of sensitivity.

5. The method according to any of claims 1 to 4, wherein the set of at least one supervised or semi-supervised machine learning method comprises a matrix factorization method allowing an identification of at least partially decorrelated matrix components, wherein determining the candidate prediction functions comprises obtaining (410) a first matrix of transcriptomic profiles from the first reference vectors;

obtaining for the matrix factorization method at least one candidate parameter set, each of said at least one candidate parameter set including a matrix component number;

for each of said at least one candidate parameter set, performing (430) the correlation analysis for the concerned candidate parameter set, wherein performing the correlation analysis for the concerned candidate parameter set comprises:

decomposing the first matrix by the matrix factorization method allowing an identification of at least partially decorrelated matrix components, wherein the number of at least partially decorrelated matrix components is equal to the matrix component number defined in the concerned candidate parameter set;

identifying, among the at least partially decorrelated matrix components, a matrix component that is the most predictive component with respect to the measures of sensitivity obtained for the tumor samples of the first batch (B1) of tumor samples corresponding respectively to the first reference vectors;

determining a candidate prediction function associated with the concerned candidate parameter set as a projection function on the most predictive component.

6. The method according to claim 5, wherein applying a candidate prediction function to a second reference vector (V2i) comprises applying (460) to the concerned second reference vector the projection function on the most predictive component to generate a predicted sensibility degree for the corresponding tumor sample of the second batch (B2) of tumor samples.

7. The method according to any of claims 1 to 6, comprising computing a sensitivity signature to the candidate therapeutic molecule as a distribution of prediction values generated by applying the selected candidate prediction function (SPF) to at least some of the second reference vectors, wherein the orientation of the scale of the prediction values is determined on the basis of the location, on the scale of the prediction values, of the prediction values of the second reference vectors associated with the highest and / or lowest measures of sensitivity.

8. Method for determining a sensitivity degree of a tumor sample of a patient to one or more candidate therapeutic molecules, said method comprising:

- obtaining (505) a vector representing a transcriptomic profile of a tumor sample of the patient;
- obtaining (510) a prediction function configured to generate a first sensitivity degree of a tumor sample to a first candidate therapeutic molecule by a method according to any of the claims 1 or 7;
- computing (520) a sensitivity degree to the first candidate therapeutic molecule as the result of the prediction function applied to the vector representing the transcriptomic profile of the tumor sample of the patient.

9. The method according to claim 8 comprising:

- obtaining (510) a prediction function configured to generate at least one second sensitivity degree of a tumor sample to at least one second candidate therapeutic molecule by a method according to any of the claims 1 or 7;
- computing (520) at least one second sensitivity degree to the respective second candidate therapeutic molecule as the result of the prediction function applied to the vector representing the transcriptomic profile of the tumor sample of the patient;
- selecting (530) a therapeutic molecule from a set comprising the first candidate therapeutic molecule and the at least one second candidate therapeutic molecule based on the comparison between the first sensitivity degree and the at least one second sensitivity degree.

10. The method according to any of the preceding claims, in which each of at least one supervised or unsupervised machine learning method is chosen from neural network methods, decision trees, k-nearest neighbors method, carrier vector machines, algorithm based on a linear model, a generalized linear model discriminant, a factor regression model, a partial least square model, a factor analysis, a support vector machine, a support vector regression, a graphical model, a tree-based model, a random forest model, a random ferns model, a naive Bayes model, a linear discriminant analysis, a quadratic linear discriminant analysis, a perceptron model, a neural network model, nearest neighbor model, a nearest prototype model, an ensemble model, a prototype-based supervised algorithm, a bagged model, a Bayesian model, a regularized linear model, a polynomial model, a rule-based model, a Gaussian process model, a mixture discriminant model, a regression spline model, a rule induction method, a prototype model, a quantile regression model, a relevance vector machine, a soft independent modelling of class analogies model, a principal component-based model, an independent-based model, a self-organizing map model.

11. The method according to any of claims 1 to 10, wherein the first and / or second reference transcriptomic profile(s) of tumor sample of human origin are obtained from patients-derived tumor xenografts, from patients-derived organoids, from patients-derived cell lines or cell culture or from patients-derived tissue.

12. The method according to any of claims 1 to 11, wherein the cancer is selected from colorectal cancer, breast cancer, lung cancer, prostate cancer, melanoma, bladder cancer, kidney cancer, neuroendocrine tumors, sarcoma, head and neck cancers, liver cancer, endometrial cancer, gastric cancer, biliary tract cancer, pancreatic ductal adenocarcinoma (PDAC), the tumor sample being a tumor sample of said cancer.

13. The method according to any of claims 1 to 12, wherein the cancer is selected from pancreatic ductal adenocarcinoma (PDAC), wherein the first batch of different tumor samples of human or animal origin is a batch of different PDAC tumor samples of human or animal origin, and wherein the second batch (B2) of different tumor samples of human or animal origin is a batch of different PDAC tumor samples of human or animal origin.

14. A computer readable medium comprising computer program instructions which when executed causes an apparatus to perform the steps of the method according to any of claims 1 to 13.

15. An apparatus comprising a computer readable medium according to claim 14 and/or means for performing the steps of the method according to any of claims 1 to 13.

310 — Obtaining first reference vectors representative of first reference transcriptomic profiles

320 — Obtaining first measures of sensitivity

330 — Correlation analysis to generate candidate prediction functions

340 — Obtaining second reference vectors representative of second reference transcriptomic profiles

350 — Obtaining second measures of sensiitivity

360 — Applying the candidate prediction functions to generate predicted sensitivity degrees

370 — Accuracy assessment to select a candidate prediction function

**Figure 1**

410 — Obtaining first matrix of reference vectors representative of first reference transcriptomic profiles

420 — Obtaining first measures of sensitivity

430 — Correlation analysis to generate candidate projection functions

440 — Obtaining second matrix of reference vectors representative of second reference transcriptomic profiles

450 — Obtaining second measures of sensitivity

460 — Applying the candidate projection functions to generate predicted sensitivity degrees

470 — Accuracy assessment to select a candidate projection function

**Figure 2**

B1

V1i

First batch of
tumor samples

D1i

First reference
vectors

First measures of
sensitivity

B2

330, 430

Correlation
analysis to
generate
candidate
prediction
functions

second batch of tumor
samples

V2i

D2i

Second reference
vectors

Second measures
of sensitivity

candidate
prediction
functions

predicted sensitivity
degrees

Assess accuracy
of prediction

PFm

PSmi

370, 470

370, 470

Select prediction
function

SPF

Selected
prediction
function

**Figure 3**

Obtain a vector representative a
transcriptomic profile of a tumor
sample of a patient — 505

Obtain a prediction function
corresponding to a candidate
therapeutic molecule — 510

Apply the prediction function to a
vector representative of a
transcriptomic profile of a patient
to generate a sensitivity degree
for the candidate therapeutic
molecule — 520

Selecting at least one candidate
therapeutic molecule based on a
comparison of
the sensitivity degrees obtained
for the candidate therapeutic
molecules — 530

**Figure 4**

**Figure 5**

**Figure 6**

**Figure 7a**

**Figure 7b**

Figure 8a

Figure 8b

**Figure 9a**

**Figure 9b**

Europäisches
Patentamt

European
Patent Office

Office européen
des brevets

## EUROPEAN SEARCH REPORT

Application Number

EP 20 30 5052

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/200410 A1 (FREENOME HOLDINGS INC [US]) 17 October 2019 (2019-10-17) * the whole document * * claims 1, 4, 12, 14, 15 * * paragraph [0242] - paragraph [0246] * * paragraph [0305] - paragraph [0319] * | 1-15 | INV. C12Q1/6886 G01N33/574 G16B25/10 G16B40/20 |
| X | WO 2018/148334 A1 (MITRA RXDX INC [US]) 16 August 2018 (2018-08-16) * the whole document * * paragraph [0112] - paragraph [0115] * | 1-15 | |
| X | US 2004/193019 A1 (WEI NIEN [US]) 30 September 2004 (2004-09-30) * the whole document * * pages 5-6, sections V-VI * | 1-15 | |
| X | CAI HUANG ET AL: "Open source machine-learning algorithms for the prediction of optimal cancer drug therapies", PLOS ONE, vol. 12, no. 10, 26 October 2017 (2017-10-26), page e0186906, XP055487702, DOI: 10.1371/journal.pone.0186906 * the whole document * | 1-15 | TECHNICAL FIELDS SEARCHED (IPC) C12Q G01N G16B |
| X | CAI HUANG ET AL: "Machine learning predicts individual cancer patient responses to therapeutic drugs with high accuracy", SCIENTIFIC REPORTS, vol. 8, no. 1, 6 November 2018 (2018-11-06), XP55671818, DOI: 10.1038/s41598-018-34753-5 * the whole document * -/-- | 1-15 | |

The present search report has been drawn up for all claims

| Place of search | Date of completion of the search | Examiner |
|---|---|---|
| The Hague | 2 June 2020 | Jenkins, Gareth |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 3

**Europäisches Patentamt**
**European Patent Office**
**Office européen des brevets**

# EUROPEAN SEARCH REPORT

Application Number

EP 20 30 5052

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ASHRAF ABOU TABL ET AL: "A Machine Learning Approach for Identifying Gene Biomarkers Guiding the Treatment of Breast Cancer", FRONTIERS IN GENETICS, vol. 10, 27 March 2019 (2019-03-27), XP55671822, DOI: 10.3389/fgene.2019.00256 * the whole document * | 1-15 | |
| Y | N KALIMUTHU ET AL: "Morphological classification of pancreatic ductal adenocarcinoma that predicts molecular subtypes andcorrelates with clinical outcome.", GUT, vol. 69, no. 2, 14 June 2019 (2019-06-14), pages 317-328, XP055671831, DOI: 10.1136/gutjnl-2019-318217 * the whole document * | 1-15 | |
| Y | GEORGIOS KAISSIS ET AL: "A machine learning algorithm predicts molecular subtypes in pancreatic ductal adenocarcinoma with differential response to gemcitabine-based versus FOLFIRINOX chemotherapy", PLOS ONE, vol. 14, no. 10, 2 October 2019 (2019-10-02), page e0218642, XP55671938, DOI: 10.1371/journal.pone.0218642 * the whole document * | 1-15 | |

TECHNICAL FIELDS SEARCHED (IPC)

-/--

The present search report has been drawn up for all claims

| Place of search | Date of completion of the search | Examiner |
|---|---|---|
| The Hague | 2 June 2020 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 20 30 5052

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Anonymous: "Supervised learning - Wikipedia", , 11 January 2020 (2020-01-11), XP55671850, Retrieved from the Internet: URL:https://en.wikipedia.org/w/index.php?title=Supervised_learning&oldid=935212464 [retrieved on 2020-02-26] * the whole document * ----- | 1-15 | |

TECHNICAL FIELDS
SEARCHED (IPC)

The present search report has been drawn up for all claims

| Place of search | Date of completion of the search | Examiner |
|---|---|---|
| The Hague | 2 June 2020 | Jenkins, Gareth |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 30 5052

This annex lists the patent family members relating to the patent documents cited in the above-mentioned European search report.
The members are as contained in the European Patent Office EDP file on
The European Patent Office is in no way liable for these particulars which are merely given for the purpose of information.

02-06-2020

| Patent document cited in search report | | | Publication date | Patent family member(s) | | | Publication date |
|---|---|---|---|---|---|---|---|
| WO 2019200410 | A1 | | 17-10-2019 | NONE | | | |
| WO 2018148334 | A1 | | 16-08-2018 | AU | 2018219831 | A1 | 12-09-2019 |
| | | | | CA | 3052987 | A1 | 16-08-2018 |
| | | | | CN | 110520539 | A | 29-11-2019 |
| | | | | EP | 3580347 | A1 | 18-12-2019 |
| | | | | JP | 2020510444 | A | 09-04-2020 |
| | | | | SG | 11201907329U | A | 27-09-2019 |
| | | | | US | 2019361006 | A1 | 28-11-2019 |
| | | | | WO | 2018148334 | A1 | 16-08-2018 |
| | | | | WO | 2018148336 | A1 | 16-08-2018 |
| US 2004193019 | A1 | | 30-09-2004 | CN | 1764837 | A | 26-04-2006 |
| | | | | TW | I287975 | B | 11-10-2007 |
| | | | | US | 2004193019 | A1 | 30-09-2004 |
| | | | | US | 2016306917 | A1 | 20-10-2016 |
| | | | | WO | 2004093822 | A2 | 04-11-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DERAMAUDT T ; RUSTGI AK ; MUTANT KRAS.** in the initiation of pancreatic cancer. *Biochim Biophys Acta,* 25 November 2005, vol. 1756 (2), 97-101
- **LECA, J. et al.** *JCI,* 2016, vol. 126, 1-17
- Nanostring: Kulkarni MM. Digital multiplexed gene expression analysis using the NanoString nCounter system. Curr Protoc Mol Biol. April 2011
- **MARIONI JC ; MASON CE ; MANE SM ; STEPHENS M ; GILAD Y.** RNA-seq: an assessment of technical reproducibility and comparison with gene expression arrays. *Genome Res.,* September 2008, vol. 18 (9), 1509-17
- **WANG Z ; GERSTEIN M ; SNYDER M.** RNA-Seq: a revolutionary tool for transcriptomics. *Nat Rev Genet.,* January 2009, vol. 10 (1), 57-63
- **STEINBOCK LJ ; RADENOVIC A.** The emergence of nanopores in next-generation sequencing. *Nanotechnology,* 2015, vol. 26 (7), 074003
- **DOBIN A ; DAVIS CA ; SCHLESINGER F ; DRENKOW J ; ZALESKI C ; JHA S ; BATUT P ; CHAISSON M ; GINGERAS TR. STAR.** ultrafast universal RNA-seq aligner. *Bioinformatics,* 01 January 2013, vol. 29 (1), 15-21
- **LIAO Y ; SMYTH GK ; SHI W.** featureCounts: an efficient general-purpose program for assigning sequence reads to genomic features. *Bioinformatics,* 2014, vol. 30 (7), 923-30
- **DERAMAUDT T ; RUSTGI AK.** Mutant KRAS in the initiation of pancreatic cancer. *Biochim Biophys Acta,* 25 November 2005, vol. 1756 (2), 97-101
- **FELDMANN et al.** Molecular genetics of pancreatic intraepithelial neoplasia. *J Hepatobiliary Pancreat Surg.,* 2007, vol. 14 (3), 224-32
- **IOVANNA et al.** Current knowledge on pancreatic cancer. *Front Oncol.,* 2012, vol. 2, 6
- **YING et al.** Genetics and biology of pancreatic ductal adenocarcinoma. *Genes Dev.,* 2016, vol. 30 (4), 355-85
- **RYAN CARR ; MARTIN E ; FERNANDEZ-ZAPICO.** Pancreatic cancer microenvironment, to target or not to target?. *EMBO Mol Med.,* February 2016, vol. 8 (2), 80-82
- **VENU G PILLARISETTY.** The pancreatic cancer microenvironment: an immunologic battleground. *OncoImmunology,* 01 August 2014, vol. 3 (8), e950171

- **J. IOVANNA ; CLOSA.** Factors released by the tumor far microenvironment are decisive for pancreatic adenocarcinoma development and progression. *Oncoimmunology,* 08 August 2017, vol. 6 (11
- **LECA, J. et al.** Cancer-associated fibroblast-derived annexin A6+ extracellular vesicles support pancreatic cancer aggressiveness. *J Clin Invest.,* 2016, vol. 126 (11), 4140-4156
- **HA BURRIS et al.** Improvements in survival and clinical benefit with gemcitabine as first-line therapy for patients with advanced pancreas cancer: a randomized trial. *J Clin Oncol.,* June 1997, vol. 15 (6), 2403-13
- **THIERRY CONROY et al.** FOLFIRINOX versus Gemcitabine for Metastatic Pancreatic Cancer. *New England Journal of Medicine,* 2011, vol. 364, 1817-1825
- **NICOLLE et al.** Pancreatic Adenocarcinoma Therapeutic Targets Revealed by Tumor-Stroma Cross-Talk Analyses in Patient-Derived Xenografts. *Cell Rep.,* 28 November 2017, vol. 21 (9), 2458-2470
- **BAILEY P et al.** Australian Pancreatic Cancer Genome Initiative. Genomic analyses identify molecular subtypes of pancreatic cancer. *Nature,* 2016, vol. 531, 47-52
- **GWEN LOMBERK et al.** Distinct epigenetic landscapes underlie the pathobiology of pancreatic cancer subtypes. *NATURE COMMUNICATIONS,* 2018, vol. 9, 1978
- **JIRI LENZ et al.** Clinicopathological correlations of nestin expression in surgically resectable pancreatic cancer including an analysis of perineural invasion. *Journal of gastrointestinal and liver diseases: JGLD,* 2011, vol. 20 (4), 389-396
- **P. VILMANN et al.** Endoscopic ultrasonography with guided fine needle aspiration biopsy in pancreatic disease. *Gastrointest Endosc.,* March 1992, vol. 38 (2), 172-3
- **PAULINE DUCONSEIL et al.** Transcriptomic Analysis Predicts Survival and Sensitivity to Anticancer Drugs of Patients with a Pancreatic Adenocarcinoma. *The American Journal of Pathology,* April 2015, vol. 185 (4
- Kulkarni MM Digital multiplexed gene expression analysis using the NanoString nCounter system. Curr Protoc Mol Biol. April 2011

- **DOBIN A ; DAVIS CA ; SCHLESINGER F ; DRENKOW J ; ZALESKI C ; JHA S ; BATUT P ; CHAISSON M ; GINGERAS TR.** STAR: ultrafast universal RNA-seq aligner. *Bioinformatics,* 01 January 2013, vol. 29 (1), 15-21
- **BULLARD et al.** Evaluation of statistical methods for normalization and differential expression in mRNA-Seq experiments. *BMC Bioinformatics,* 2010, vol. 11, 94, http://www.biomedcentral.com/1471-2105/11/94
- **MOFFITT RA ; MARAYATI R ; FLATE EL et al.** Virtual microdissection identifies distinct tumor- and stroma-specific subtypes of pancreatic ductal adenocarcinoma. *Nat Genet,* 2015, vol. 47, 1168-1178
- **MAURER HC ; HOLMSTROM SR ; HE J et al.** Experimental microdissection enables functional harmonisation of pancreatic cancer subtypes. *Gut,* 2019